# EUROPEAN PATENT APPLICATION

(11) **EP 3 889 259 A1**
(43) Date of publication of application: **06.10.2021**
(21) Application number: 20166567.6
(22) Date of filing: 30.03.2020
(51) Int. Cl.: C12N 15/11

(54) **INTERNAL STANDARD FOR CRISPR GUIDE RNA**

(71) Applicant: IMBA-Institut für Molekulare Biotechnologie GmbH, 1030 Wien (AT)
(72) Inventor: ELLING, Ulrich, 2380 Perchtoldsdorf (AT); SELL, Annika, 1060 Wien (AT); UIJTTEWAAL, Esther C.H., 1030 Wien (AT)
(74) Representative: SONN Patentanwälte OG

(57) **Abstract**

The present invention provides a nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein the guide disruption sequence is not flanked by the second pair of recombinase recognition sites and wherein the sequences flanked by the first and second recombinase recognition sites overlap; methods of using such a sgRNA, transgenic cells and kits.

SEQUENCE LISTING

<110>  IMBA - INSTITUT FÜR MOLEKULARE BIOTECHNOLOGIE GMBH

<120>  Internal standard for CRISPR guide RNA

<130>  r75930

<160>  20

<170>  PatentIn version 3.5

<210>  1
<211>  62
<212>  DNA
<213>  Artificial Sequence

<220>
<223>  primer

<400>  1
aatgatacgg cgaccaccga gatctacaca gataacgagg gcctatttcc catgattcct      60

tc                                                                      62

<210>  2
<211>  62
<212>  DNA
<213>  Artificial Sequence

<220>
<223>  primer

<400>  2
aatgatacgg cgaccaccga gatctacaca gcttgcgagg gcctatttcc catgattcct      60

tc                                                                      62

<210>  3
<211>  62
<212>  DNA
<213>  Artificial Sequence

<220>
<223>  primer

<400>  3
aatgatacgg cgaccaccga gatctacaca ggacacgagg gcctatttcc catgattcct      60

tc                                                                      62

<210>  4
<211>  62
<212>  DNA
<213>  Artificial Sequence

<220>
<223>  primer

<400>  4

aatgatacgg cgaccaccga gatctacaca tcactcgagg gcctatttcc catgattcct 60

tc 62

<210> 5
<211> 62
<212> DNA
<213> Artificial Sequence

<220>
<223> primer

<400> 5
aatgatacgg cgaccaccga gatctacacc aacaccgagg gcctatttcc catgattcct 60

tc 62

<210> 6
<211> 62
<212> DNA
<213> Artificial Sequence

<220>
<223> primer

<400> 6
aatgatacgg cgaccaccga gatctacacc acgcccgagg gcctatttcc catgattcct 60

tc 62

<210> 7
<211> 62
<212> DNA
<213> Artificial Sequence

<220>
<223> primer

<400> 7
aatgatacgg cgaccaccga gatctacacc attaccgagg gcctatttcc catgattcct 60

tc 62

<210> 8
<211> 62
<212> DNA
<213> Artificial Sequence

<220>
<223> primer

<400> 8
aatgatacgg cgaccaccga gatctacacc cccaacgagg gcctatttcc catgattcct 60

tc 62

<210> 9
<211> 62

<212>  DNA
<213>  Artificial Sequence

<220>
<223>  primer

<400>  9
aatgatacgg cgaccaccga gatctacacc gtcatcgagg gcctatttcc catgattcct      60

tc                                                                      62


<210>  10
<211>  62
<212>  DNA
<213>  Artificial Sequence

<220>
<223>  primer

<400>  10
aatgatacgg cgaccaccga gatctacacc tatgccgagg gcctatttcc catgattcct      60

tc                                                                      62


<210>  11
<211>  62
<212>  DNA
<213>  Artificial Sequence

<220>
<223>  primer

<400>  11
aatgatacgg cgaccaccga gatctacacc tccgccgagg gcctatttcc catgattcct      60

tc                                                                      62


<210>  12
<211>  62
<212>  DNA
<213>  Artificial Sequence

<220>
<223>  primer

<400>  12
aatgatacgg cgaccaccga gatctacact gccgacgagg gcctatttcc catgattcct      60

tc                                                                      62


<210>  13
<211>  62
<212>  DNA
<213>  Artificial Sequence

<220>
<223>  primer

<400>  13

aatgatacgg cgaccaccga gatctacact gtagacgagg gcctatttcc catgattcct 60

tc 62


<210> 14
<211> 62
<212> DNA
<213> Artificial Sequence

<220>
<223> primer

<400> 14
aatgatacgg cgaccaccga gatctacact tgccacgagg gcctatttcc catgattcct 60

tc 62


<210> 15
<211> 39
<212> DNA
<213> Artificial Sequence

<220>
<223> primer

<400> 15
caagcagaag acggcatacg agataccgtt gatgagtag 39


<210> 16
<211> 44
<212> DNA
<213> Artificial Sequence

<220>
<223> primer

<400> 16
cgatttcttg gctttatata tcttgtggaa aggacgaaac accg 44


<210> 17
<211> 62
<212> DNA
<213> Artificial Sequence

<220>
<223> primer


<220>
<221> misc_feature
<222> (30)..(35)
<223> n is a, c, g, or t

<400> 17
aatgatacgg cgaccaccga gatctacacn nnnnncgagg gcctatttcc catgattcct 60

tc 62

<210> 18
<211> 1595
<212> DNA
<213> Artificial Sequence

<220>
<223> sgRNA construct sequence

<400> 18
gcatatacga tacaaggctg ttagagagat aattggaatt aatttgactg taaacacaaa 60

gatattagta caaaatacgt gacgtagaaa gtaataattt cttgggtagt ttgcagtttt 120

aaaattatgt tttaaaatgg actatcatat gcttaccgta acttgaaagt atttcgattt 180

cttggcttta tatatcttgt ggaaaggacg aaacaccggg tcttcgctcg agagaagacc 240

tgtttaagag ctatgctgat aacttcgtat agcatacatt atacgaagtt attttttttt 300

tttgcggccg cccagaatta taacttcgta taatgtgtac tatacgaagt tattaattaa 360

ttctggcctg caggaattct accgggtagg ggaggcgctt ttcccaaggc agtctggagc 420

atgcgcttta gcagccccgc tgggcacttg gcgctacaca agtggcctct ggcctcgcac 480

acattccaca tccaccggta ggcgccaacc ggctccgttc tttggtggcc ccttcgcgcc 540

accttctact cctcccctag tcaggaagtt cccccccgcc ccgcagctcg cgtcgtgcag 600

gacgtgacaa atggaagtag cacgtctcac tagtctcgtg cagatggaca gcaccgctga 660

gcaatggaag cgggtaggcc tttggggcag cggccaatag cagctttgct ccttcgcttt 720

ctgggctcag aggctgggaa ggggtgggtc cgggggcggg ctcaggggcg ggctcagggg 780

cggggcgggc gcccgaaggt cctccggagg cccggcattc tgcacgcttc aaaagcgcac 840

gtctgccgcg ctgttctcct cttcctcatc tccgggcctt tcgacctgca gccaatatgg 900

ccaagccttt gtctcaagaa gaatccaccc tcattgaaag agcaacggct acaatcaaca 960

gcatccccat ctctgaggat tacagcgtcg ccagcgcagc tctctctagc gacggccgca 1020

tcttcactgg tgtcaatgta tatcatttta ctgggggacc ttgtgcagaa ctcgtggtgc 1080

tgggcactgc tgctgctgcg gcagctggca acctgacttg tatcgtcgcg atcggaaatg 1140

agaacagggg catcttgagc ccctgcggac ggtgccgaca ggtgcttctc gatctgcatc 1200

ctgggatcaa agccatagtg aaggacagtg atggacagcc gacggcagtt gggattcgtg 1260

aattgctgcc ctctggttat gtgtgggagg gctgacctgc aggataactt cgtatagcat 1320

acattatacg aagttatcag catagcaagt ttaaataagg ctagtccgtt atcaacttga 1380

aaaagtggca ccgagtcggt gcttttttgt tttagagcta gaaatagcaa gttaaaataa 1440

ggctagtccg tttttagcgc gtgcgccaat tctgcagaca aatggctcta gcataacttc 1500

gtataatgtg tactatacga agttatctag aggtacccgt taagatcgga agagcacacg 1560

tctgaactcc agtcacaaaa ttatctcgta tgccg 1595

<210>  19
<211>  1668
<212>  DNA
<213>  Artificial Sequence

<220>
<223>  sgRNA construct sequence

<400>  19
gcatatacga tacaaggctg ttagagagat aattggaatt aatttgactg taaacacaaa      60

gatattagta caaaatacgt gacgtagaaa gtaataattt cttgggtagt ttgcagtttt     120

aaaattatgt tttaaaatgg actatcatat gcttaccgta acttgaaagt atttcgattt     180

cttggcttta tatatcttgt ggaaaggacg aaacaccggg tcttcgctcg agagaagacc     240

tgtttaagag ctatgctgat aacttcgtat agcatacatt atacgaagtt attttttttt     300

tttgcggccg ccaagagagc agaaatagca agtaactcat taggtaaata aggtagtccg     360

tttagtgccg tagccaattc tcagacattt aatggctcta aacgtaacat ttacaaatta     420

taacttcgta taatgtgtac tatacgaagt tattaattaa ttctggttcg aacctgataa     480

cttcgtatag catacattat acgaagttat cattcgaatg tagaattcta ccgggtaggg     540

gaggcgcttt tcccaaggca gtctggagca tgcgctttag cagccccgct gggcacttgg     600

cgctacacaa gtggcctctg gcctcgcaca cattccacat ccaccggtag gcgccaaccg     660

gctccgttct ttggtggccc cttcgcgcca ccttctactc ctcccctagt caggaagttc     720

ccccccgccc cgcagctcgc gtcgtgcagg acgtgacaaa tggaagtagc acgtctcact     780

agtctcgtgc agatggacag caccgctgag caatggaagc gggtaggcct ttggggcagc     840

ggccaatagc agctttgctc cttcgctttc tgggctcaga ggctgggaag gggtgggtcc     900

gggggcgggc tcaggggcgg gctcaggggc ggggcgggcg cccgaaggtc ctccggaggc     960

ccggcattct gcacgcttca aaagcgcacg tctgccgcgc tgttctcctc ttcctcatct    1020

ccgggccttt cgacctgcag ccaatatggc caagcctttg tctcaagaag aatccaccct    1080

cattgaaaga gcaacggcta caatcaacag catccccatc tctgaggatt acagcgtcgc    1140

cagcgcagct ctctctagcg acggccgcat cttcactggt gtcaatgtat atcattttac    1200

tgggggacct tgtgcagaac tcgtggtgct gggcactgct gctgctgcgg cagctggcaa    1260

cctgacttgt atcgtcgcga tcggaaatga gaacaggggc atcttgagcc cctgcggacg    1320

gtgccgacag gtgcttctcg atctgcatcc tgggatcaaa gccatagtga aggacagtga    1380

tggacagccg acggcagttg ggattcgtga attgctgccc tctggttatg tgtgggaggg    1440

ctgacctgca ggataacttc gtatagcata cattatacga agttatcagc atagcaagtt    1500

taaataaggc tagtccgtta tcaacttgaa aaagtggcac cgagtcggtg cttttttgtt    1560

ttagcataac ttcgtataat gtgtactata cgaagttatc tagaggtacc cgttaagatc    1620

ggaagagcac acgtctgaac tccagtcaca aaattatctc gtatgccg 1668

<210> 20
<211> 1622
<212> DNA
<213> Artificial Sequence

<220>
<223> sgRNA construct sequence

<400> 20
gcatatacga tacaaggctg ttagagagat aattggaatt aatttgactg taaacacaaa 60

gatattagta caaaatacgt gacgtagaaa gtaataattt cttgggtagt ttgcagtttt 120

aaaattatgt tttaaaatgg actatcatat gcttaccgta acttgaaagt atttcgattt 180

cttggcttta tatatcttgt ggaaaggacg aaacaccggg tcttcgctcg agagaagacc 240

tgtttaagag ctatgctgat aacttcgtat agcatacatt atacgaagtt attttttttt 300

tttgcggccg ccaagagagc agaaatagca agtaactcat taggtaaata aggtagtccg 360

tttagtgccg tagccaattc tcagacattt aatggctcta aacgtaacat ttacaaatta 420

taacttcgta taatgtgtac tatacgaagt tattaattaa ttctggttcg aatgtagaat 480

tctaccgggt aggggaggcg cttttcccaa ggcagtctgg agcatgcgct ttagcagccc 540

cgctgggcac ttggcgctac acaagtggcc tctggcctcg cacacattcc acatccaccg 600

gtaggcgcca accggctccg ttctttggtg gccccttcgc gccaccttct actcctcccc 660

tagtcaggaa gttccccccc gccccgcagc tcgcgtcgtg caggacgtga caaatggaag 720

tagcacgtct cactagtctc gtgcagatgg acagcaccgc tgagcaatgg aagcgggtag 780

gcctttgggg cagcggccaa tagcagcttt gctccttcgc tttctgggct cagaggctgg 840

gaaggggtgg gtccgggggc gggctcaggg gcgggctcag gggcggggcg ggcgcccgaa 900

ggtcctccgg aggcccggca ttctgcacgc ttcaaaagcg cacgtctgcc gcgctgttct 960

cctcttcctc atctccgggc ctttcgacct gcagccaata tggccaagcc tttgtctcaa 1020

gaagaatcca ccctcattga aagagcaacg gctacaatca acagcatccc catctctgag 1080

gattacagcg tcgccagcgc agctctctct agcgacggcc gcatcttcac tggtgtcaat 1140

gtatatcatt ttactggggg accttgtgca gaactcgtgg tgctgggcac tgctgctgct 1200

gcggcagctg gcaacctgac ttgtatcgtc gcgatcggaa atgagaacag gggcatcttg 1260

agcccctgcg gacggtgccg acaggtgctt ctcgatctgc atcctgggat caaagccata 1320

gtgaaggaca gtgatggaca gccgacggca gttgggattc gtgaattgct gccctctggt 1380

tatgtgtggg agggctgacc tgcaggataa cttcgtatag catacattat acgaagttat 1440

cagcatagca agtttaaata aggctagtcc gttatcaact tgaaaaagtg gcaccgagtc 1500

ggtgcttttt tgttttagca taacttcgta taatgtgtac tatacgaagt tatctagagg 1560

tacccgttaa gatcggaaga gcacacgtct gaactccagt cacaaaatta tctcgtatgc 1620

cg 1622

## Description

### Background of the invention

CRISPR screening has become the prime method to functionally interrogate the genome in various assays. In positive selection screens the enrichment of sgRNAs in the cell population is examined to identify the genes that, upon knockout, enhance the cells survival. Whereas in negative selection screening certain sgRNAs will be depleted from the cell population, as the knockout of the corresponding genes will result in cell death (Miles et al. FEBS J. 283, 2016: 3170-3180). These screens are also called essentialome screens. To do so, cell lines expressing the bacterial endonuclease Cas9 are transduced with sgRNA libraries to induce loss of function mutations in genes. sgRNAs are short RNAs consisting of a 20 bp gene-specific stretch as well as a 3' scaffold that guides a Cas enzyme to genomic loci complementary to the sgRNA sequence. Upon binding, Cas will induce genetic or regulatory changes.

For high quality assessment of gene function multiple independent cells must be transduced with a specific sgRNA to account for cellular heterogeneity as well as various editing outcomes. In pooled genetic screens, the number of independently targeted cells is typically maintained above 300-1,000 cells/sgRNA. Thus, if each gene is targeted with five sgRNAs in a genome wide (20,000 genes) screening approach, this amounts to a minimum screen size of 300^{∗}5^{∗}20,000 = 30 million cells throughout the experiment. E.g. Wang et al. (Science 343, 2014: 80-84) describes a large scale CRISPR-Cas screen using 73,000 sgRNAs to transduce 90 million target cells, i.e. 1,233 cells/sgRNA. 5-10 sgRNAs/gene are recommended. Such screens essentially work with high numbers of highly viable immortalized cells.

This requirement of high numbers of cells is hard to accommodate in some experiments, such as with primary cell lines having heterogenous growth, with organoids of limited size as well as with *in vivo* screens. Therefore, there is a need for a robust method that is able to reduce the number of required cells and to overcome cell growth bottlenecks in screens.

### Summary of the invention

The present invention provides a nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein the guide disruption sequence is not flanked by the second pair of recombinase recognition sites and/or wherein the second pair of recombinase recognition sites flank a part of the sgRNA that is required to form an active sgRNA; and wherein the sequences flanked by the first and second recombinase recognition sites overlap.

Related thereto the invention provides a nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein one recombinase recognition site of the second recombinase recognition site pair is located between the first pair of recombinase recognition sites and optionally downstream of the guide disruption sequence, and another recombinase recognition site of the second recombinase recognition site pair is located downstream of the first pair of recombinase recognition sites.

The present invention further provides a method of expressing an sgRNA of the CRISPR/Cas system upon recombinase stimulation, comprising A) providing a plurality of cells with a plurality of sgRNA-encoding nucleic acids of the invention, B) introducing or activating one or more recombinases in the cells that are capable of activating the first and second recombinase recognition site pairs, C) wherein activation of the first recombinase recognition site pair and of the second recombinase recognition site pair are competing reactions, wherein activation of the first recombinase recognition site pair leads to expression of an active sgRNA and wherein activation of the second recombinase recognition site pair inactivates the sgRNA sequence.

Further provided is a cell, comprising the nucleic acid comprising a sequence encoding a single guide RNA (sgRNA) of a CRISPR/Cas system of the invention. Even further provided is a kit comprising i) a nucleic acid encoding the sgRNA and ii) a nucleic acid for expression of a recombinase that activates a recombinase recognition site pair of the sgRNA-encoding nucleic acid.

All embodiments of the invention are described together in the following detailed description and all preferred embodiments relate to all embodiments, aspects, nucleic acids, methods, cells and kits alike. E.g. descriptions of nucleic acids, cells and kits as such also apply to the nucleic acids and means used in the inventive methods. Preferred and detailed descriptions of the inventive methods apply alike to suitability's and requirements of the inventive nucleic acids, cells, kits or products in general, like the expressed sgRNA. All embodiments can be combined with each other, except where otherwise stated.

### Detailed description of the invention

A major challenge for high resolution *in vivo* CRISPR methods is the representation of each sgRNA in multiple independent cells (Miles et al., *supra*). Ideally a gene is targeted by multiple sgRNAs, 5-10 sgRNAs/gene and each sgRNA is presented in 300-1,000 cells. This so-called library complexity is easy to achieve and maintain in *in vitro* immortalized cell lines. However, to achieve this complexity in primary cells or *in vivo* is much more difficult and thus far impossible for genome wide libraries. Furthermore, growth bottlenecks, such as in selection steps that remove high numbers of cells from the system, cause losses in complexity so that the screen quality suffers due to underrepresented or lost sgRNAs. Further bottlenecks of representation, include: i) Infection efficiency: how many sgRNAs are successfully transduced into independent cells. Some cells are more difficult to infect than others. Inefficient sgRNA infection leads to clonal outgrowth and a loss of many sgRNAs of the library before screening has started. ii) Cell availability: the amount of cells that can be expanded to achieve high library complexity. As some cell lines have limited growth abilities it is difficult to have enough sgRNAs represented prior to actual screening, and also to be maintained during screening. iii) Engraftment: the amount of the transduced (e.g. tumor) cells that survive after injection *in vivo,* is also dependent on the place of cell injection. Depending on which cells will engraft, only a limited amount of sgRNAs will be represented. iv) Differentiation: the bias of certain cells to differentiate instead of others in the population. Together, these factors contribute to an extremely wide, stochastic spread of sgRNA representation in *in vivo* experiments independent of any biological activity. Therefore, absolute representation is no useful predictor for any phenotype induced by a specific sgRNA and will result in poor validation of screen results.

Besides those bottlenecks, also cellular heterogeneity plays a pivotal role in confusing and sometimes even contradictory screening results. Within a cell population there will be often some cells that acquired viability advantages, but may also happen after genetic editing e.g. by addition of reporters or in immunofluorescent assays.

The present invention provides a CRISPR system that is able to reduce the number of needed cells at the point of transfection or of surviving cells in bottlenecks. The inventive method is based on a stochastic activation or inactivation of the sgRNA, thereby creating both activated and inactivates sgRNAs in a population of cells. The inactivated sgRNA can act as control to the activated sgRNA or vice-versa. Importantly, the time of activation and inactivation can be controlled - and is usually done after any such bottleneck -, thereby allowing the control to be created at the same point as the test sgRNA species, such as when cell numbers have recovered in a growth phase, thereby bypassing the bottleneck or the effects of cellular heterogeneity.

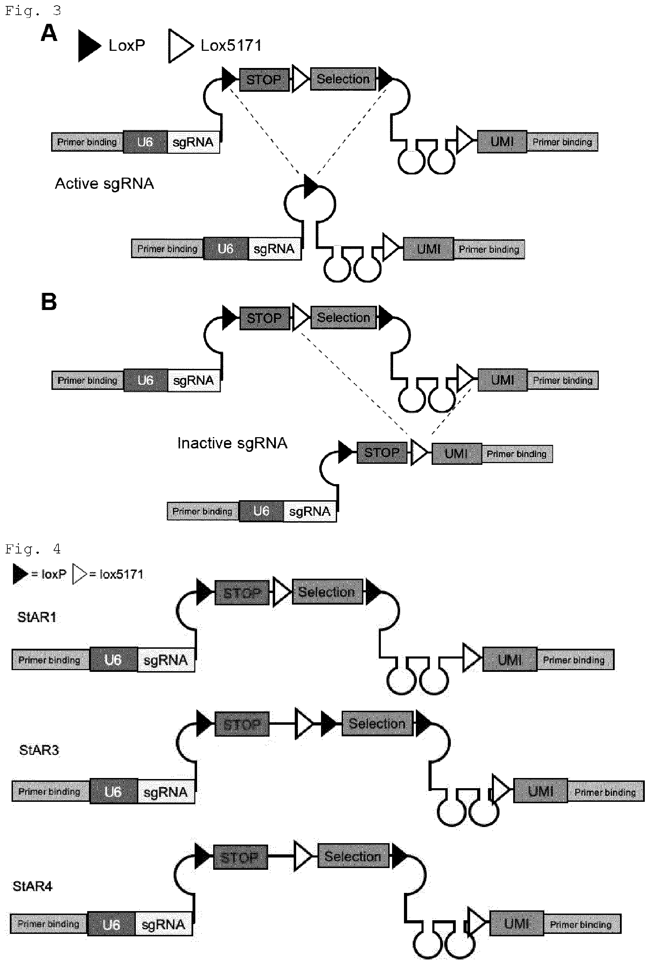

Based on the stochastic activity, the inventive method is also referred to as CRISPR-StAR, Stochastic Activation by Recombination. By the usage of a recombination system, it is possible to express sgRNAs in an active or inactive state. By alternative recombinations at two different pairs or sets of recombinase recognition sites, either activation or inactivation occurs, generating an internal control in the cell population, e.g. usually the inactive sgRNA (Figure 3).

For such a conditional expression of a single guide RNA (sgRNA) of a CRISPR/Cas system, the invention provides a nucleic acid, such as an expression cassette, that comprises a sequence that encodes a sgRNA sequence. Within the sgRNA, recombinase recognition sites are placed that allow the inventive activation or inactivation. Recombinase recognition sites in sgRNAs have been previously disclosed in WO 2017/158153 A1 and Chylinski et al, Nature Communications 10, 2019: 5454, termed CRISPR-Switch; both references incorporated herein by reference. The invention utilizes the basic principle of recombinase usage in sgRNA modification and takes this principle several steps further to provide a conditional activation/inactivation system using at least two different pairs or sets of recombinase recognition to overcome the problem of inadequate representation in low cell number situations.

sgRNAs are RNAs used in a CRISPR/Cas method, such as CRISPRi or CRISPRa, in combination with a Cas enzyme, like Cas1, Cas2, Cas3, Cas9, dCas9, Cas10 or Cas12a. A single guide RNA (sgRNA) comprises both the crRNA (CRISPR RNA) and tracrRNA (transactivating crRNA) as a single construct. The crRNA is also referred to as guideRNA for containing the DNA guiding sequence. The tracrRNA and the crRNA can be linked to form a single molecule, i.e. the single guide RNA (sgRNA). tracrRNA and crRNA hybridize in a complementary region. This complementary region can be used for the linkage and may form, together with a linkage, a stem-loop, called the crRNA:tracrRNA stem loop herein. Since this region in most cases mediates binding to a Cas protein, it may also be referred to as Cas binding element. Site-specific cleavage occurs at locations determined by both base-pairing complementarity between the crRNA and the target protospacer DNA, and a short motif [referred to as the protospacer adjacent motif (PAM)] juxtaposed to the complementary region in the target DNA. The target DNA may be in any DNA molecule that should be modified. It may be of a gene that shall be modified. A typical use of the CRISPR/Cas system is to introduce mutations or modifications on DNA or alter gene expression. The design of sgRNAs is by now conventional, as reviewed e.g. by Ciu et al. (Interdisciplinary Sciences Computational Life Sciences 2018, DOI: 10.1007/s12539-018-0298-z) or Hwang et al. (BMC Bioinformatics 19, 2018:542). Many tools exist that can be used according to the invention to generate a sgRNA sequence targeting a gene of interest, having an activity of interest, e.g. activation or inhibition of a gene by action of CRISPR/Cas.

According to the invention, the sgRNA sequence is interrupted by a guide disruption sequence. This guide disruption sequence prevents formation of an active sgRNA that can be used by a Cas enzyme. The guide disruption sequence is flanked by a first pair of recombinase recognition sites, which makes the guide disruption sequence deletable by recombinase action on these sites. The sgRNA sequence comprises a second pair of recombinase recognition sites that has a different recombinase recognition sequence than the first pair of recombinase recognition sites. The difference from the fist pair of recombinase recognition sites means that no recombination mixture or connection between the two types of recombinase recognition sites occurs. Different recombinases may be used to this effect, but it is also possible to use the same recombinase for the first and second sites because some recombinases, like Cre, recognize many sites without connecting such different sites during recombination.

Of note, the first and second recombinase recognitions sites both effect deletion upon recombination, i.e. they are in the same orientation (as opposed to in opposite orientation, which would lead to sequence inversions).

A major difference between the first and second recombinase recognition sites is that only the first pair of sites flanks the guide disruption sequence, whereas the second pair does not flank the guide disruption sequence. This means that recombination at the first pair removes the guide disruption sequence (turning the sgRNA active), whereas recombination at the second pair does not (the sgRNA remains inactive). Herein, a reference to "inactivating the sgRNA sequence" means that the sgRNA is turned inactive so that it cannot give rise to the active sgRNA anymore, i.e. the recombination at the second recombinase recognition site pair. In addition, sequences flanked by the first and by the second pair overlap, causing recombination of the first and second pair to be mutually exclusive because recombination at the first pair (thereby deleting its flanked sequence) will remove a required recombinase recognition site of the second pair and in the other case, recombination at the second pair (thereby deleting its flanked sequence) will remove a required recombinase recognition site of the first pair. Accordingly, in the inventive sgRNA, one recombinase recognition site of the second recombinase recognition site pair is located between the first pair of recombinase recognition sites (and preferably downstream of the guide disruption sequence), and another recombinase recognition site of the second recombinase recognition site pair is located downstream of the first pair of recombinase recognition sites. "Downstream" means in 5' to 3' direction on the sequence of the sgRNA. In other words, the inventive nucleic acid can also be defined as a nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein the guide disruption sequence is not flanked by the second pair of recombinase recognition sites and wherein the sequences flanked by the first and second recombinase recognition sites overlap. The mentioned second recombinase recognition site that is located between the first pair of recombinase recognition sites is optionally and preferably also downstream of the guide disruption sequence. This would leave a guide disruption sequence outside the region flanked by the pair of second recombinase recognition sites and thus upon inactivation leave the guide disruption sequence in effect. This efficiently produces an inactivated sgRNA. However other options exist, such as removing parts of the tracrRNA that would be required to form an active sgRNA. Such a removal would also inactivate the sgRNA. This removal of parts of the tracrRNA can of course be combined with locating the guide disruption sequence outside the region that is flanked by the pair of second recombinase recognition sites. Thus, in an alternative to "the guide disruption sequence is not flanked by the second pair of recombinase recognition sites" it is also possible to provide a nucleic acid wherein the second pair of recombinase recognition sites flank a part of the sgRNA that is required to form an active sgRNA, such as an essential tracrRNA part as mentioned above. Such an essential part of the tracrRNA could be a Cas-binding element or a part of tracrRNA that is required for any function of tracrRNA as described herein. This option with a second recombinase recognition site downstream of the guide disruption sequence and one of the first pair of recombinase recognition sites particularly applies to sgRNAs with the 5'-to-3' structure of a guide followed by the tracr parts. Some Cas enzymes recognize a different order, such as when the guide is downstream (3' side) of the tracr. For these Cas enzymes, the order in the sgRNA is reversed and the second recombinase recognition site should be upstream (i.e. in 3' to 5' direction) of the guide disruption sequence. Accordingly, the invention also provides a nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein one recombinase recognition site of the second recombinase recognition site pair is located between the first pair of recombinase recognition sites (and preferably upstream of the guide disruption sequence), and another recombinase recognition site of the second recombinase recognition site pair is located upstream of the first pair of recombinase recognition sites.

Based on this sequence structure only one pair of said first or second recombinase recognition sites can lead to a recombination reaction resulting in a deletion of the sequence between the recombinase recognition sites. Which one of the pair of sites, i.e. the first one or the second, results in recombination ("selection") is essentially stochastic. It is possible to select recombination sequences that are preferred over others but essentially, the site selection by the recombinase remains stochastic. Briefly, recombination at shorter flanked sequences is usually preferred by recombinase enzymes over longer flanked sequences, as is explained - together with other options - in more detail below. When using a population or plurality of cells with the sgRNA sequences of the invention, the stochastic recombinase site selection by the recombinase enzyme means that a (first) group of the cells will have the first recombinase recognition site recombination (activation of sgRNA) and another (second) group of the cells will have the second recombinase recognition site recombination (inactivation of sgRNA). The ratio of the first group of cells to the second group of cells depends on the selection preference of recombination between the first pair and second pair of recombinase recognition sites.

According to these principles, the present invention provides a method of expressing an sgRNA of the CRISPR/Cas system upon recombinase stimulation, comprising:
A) providing a plurality of cells with a plurality of nucleic acids encoding the sgRNA of the invention,
B) introducing or activating one or more recombinases in the cells that are capable of activating the first and second recombinase recognition site pairs,
C) wherein activation of the first recombinase recognition site pair and of the second recombinase recognition site pair are competing reactions, wherein activation of the first recombinase recognition site pair leads to expression of an active sgRNA and wherein activation of the second recombinase recognition site pair inactivates the sgRNA sequence.

One advantage of the invention is that the time of recombinase introduction or activation can be selected by the practitioner. So, it is possible to let the cells grow to a desired number which is best for the assay or screening method that is considered. This allows to choose beneficial time-points for recombination, such as upon a desired differentiation stage (e.g. last or terminal differentiation) in differentiation paradigms when starting with toti-, pluri-, or multipotent (stem) cells. On the other hand, the presence of both active and inactive sgRNAs provides for an internal control within the cell population so that even with low and sub-optimal cell numbers an improvement is achieved.

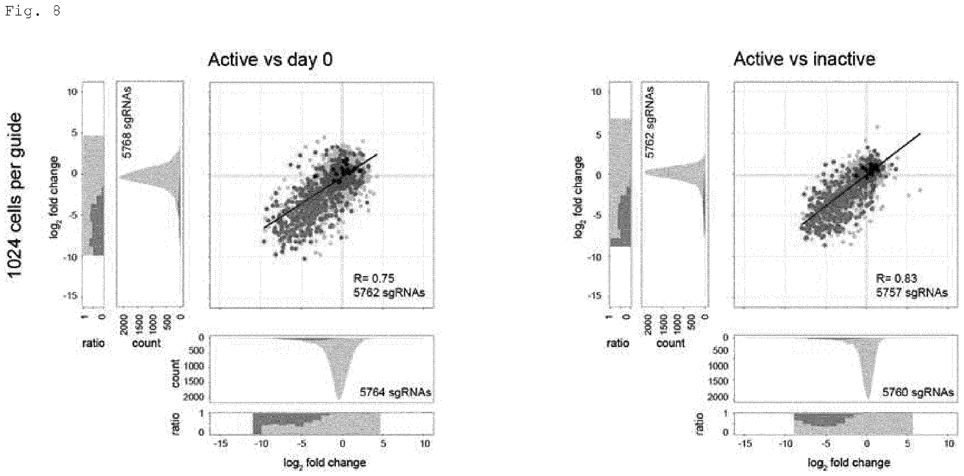

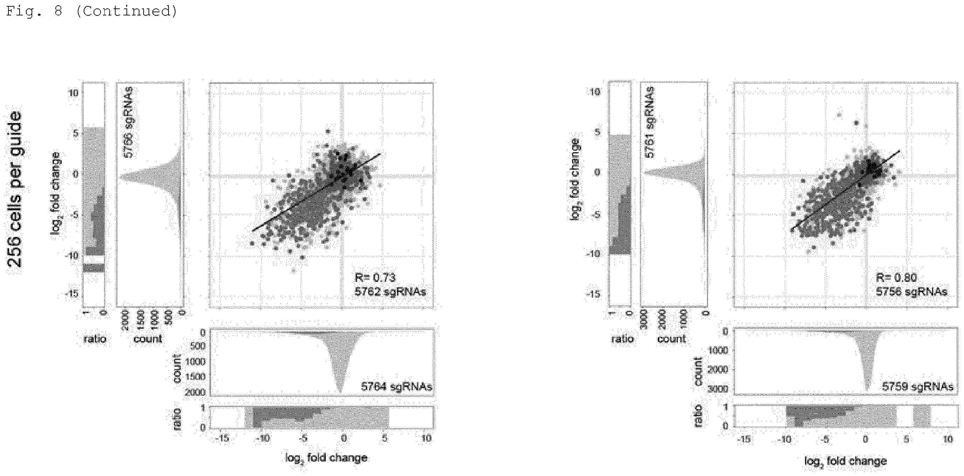

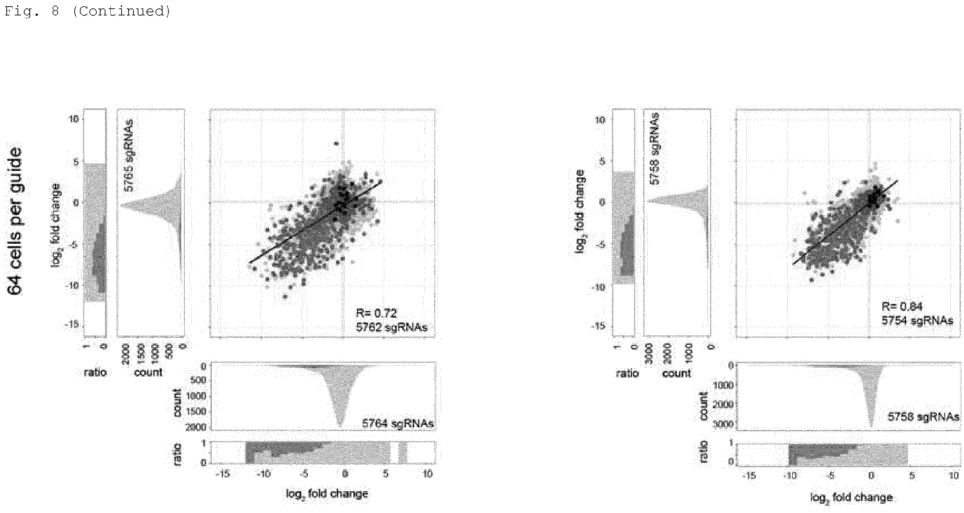

The inventive CRISPR-StAR method can circumvent the stochastic representative drift by comparison of sgRNA abundance to the internal inactive controls as opposed to the sgRNA abundance before the bottleneck. Especially for screens that go through a bottleneck of sgRNA representation, this control method is more robust than the conventional manner to control sgRNA presents before and after screening (Figures 8-13). CRISPR-StAR reduces the noise and enables population separation of essential sgRNAs and control sgRNAs even with low cell numbers.

To maintain comparability of the active and inactive sgRNA, it is essential that both, i.e. also the inactive sgRNA maintains at least a part of the guide sequence (corresponding to crRNA, see above) so that the inactive sgRNA can be assigned to an active sgRNA. Of course, also other sequences can be used to assign inactive and active sgRNAs that both stem from the same nucleic acid, to one another, when said sequence is preserved in both recombination events and is unique to the sgRNA so that it is not confused with other sgRNA sequences that have other genetic targets.

In preferred embodiments of the invention, the cells with the inactive part of the sgRNA sequence are identified to detect the presence of a sgRNA sequence. Using the inactive sgRNA as means for detecting the presence of a sgRNA in an experiment allows identifying a sgRNA (especially its guide sequence) that was present and thus has been tested in an experiment irrespective of any losses due to bottlenecks or other reasons for absences. Absence of an active sgRNA in a cell population usually does not allow such a conclusion, because its absence may also be caused by the activity of the sgRNA itself, e.g. when detrimental for cell survival. This means that absence of an active sgRNA (as in prior art) can mean that either the sgRNA prevents cell survival (and hence sgRNA detection) or that it was lost during the experiment. Absence of the inventive inactive sgRNA most likely means that it was lost in the experiment but since the sgRNA remained inactive it would not have an effect on cell survival and thus this reason can be ruled out. This means that the inventive system provides evidence of absence of a result (of cell survival).

The inventive system allows screening at lower representation than initially required for large-scale screening and will therefore overcome the bottlenecks. This is particularly beneficial for genetic screens *in vivo,* especially large-scale genetic screens.

To overcome cell effects of low cell number in cell survival or growth bottlenecks, it is preferred to let the cells with the inventive nucleic acid grow to a desirable number before the recombinase is (actively) introduced or activated. For example, in preferred embodiments of the invention, the cells are multiplied (cloned) after step A) and before step B), preferably wherein the cells are multiplied to a number of at least 250, preferably at least 300, at least 350 or at least 400, cells per number of different sgRNA sequences used in an experiment. Also higher numbers are possible and desired in some cases, such as at least 500 or at least 800, e.g. 500 to 5000 or 800 to 2000, cells per number of different sgRNA sequences of the invention, or more. Due to cell heterogeneity, many cells are tested in parallel in a CRISPR experiment. The inventive method and the means for it allow to generate these cells with the inventive nucleic acid in them (after any steps that may reduce the cell number, like transfection, grafting, etc.) and then activating the recombinase so that recombination at either the first or second recombinase recognition sites takes place, thereby activating or inactivating the sgRNA. After this activation/recombinase action, the genetic or physiological effects of the sgRNA in a cell or in an organism can be observed. The expression "capable of activating the first and second recombinase recognition site pairs" refers to a recombinase that is capable to cause recombination at the first and second recombinase recognition sites. "Activating a recombinase" means that the recombinase performs the recombination. A recombinase may exist in an inactive form and turn active once a cofactor or other activation factor is present.

In preferred embodiments, the recombinase is an inducible recombinase. This allows easier preparation of transgenic cells, with a recombinase being present, which then can be activated later as described above in step B). Inducible recombinases may be inducible by using an inducible promoter or transcription enhancer. Activating the promoter or enhancer leads to recombinase expression and activity. Another example is a recombinase that is (as a protein) inactive and activated by action of an activator. Such recombinase may be genetically engineered. One example is CreER, a Cre recombinase that is fused to an estrogen receptor (ER) or a (mutated) ligand-binding domain of the ER. By providing a ligand to the estrogen receptor or the domain, such as 4OH-tamoxifen or tamoxifen, the Cre enzyme is activated. Further methods include conditional gene expression systems such as doxycycline-dependent or light-induced expression of Cre or Flp recombinases. Similarly, cell type or stage specific promoters can be used to induce gene expression at certain time or location. Yet another example may be chemical stabilization (shield) or destabilisation (degron) of said recombinase activity.

For example, the recombinase can be induced or activated (e.g. by administering 4OH-tamoxifen) in a cell or cell culture or in an animal that comprises cells with the inventive nucleic acid, after a bottleneck of cell/sgRNA representation, e.g. when cells have recovered to, for example, at least 500 or at least 1,000 cells/sgRNA.

Preferably the inventive nucleic acid is used in a cell, i.e. it is or has been provided to the cells in the inventive methods. The cells should also be able to stably propagate the nucleic acid with the cell's proliferation. This can e.g. be done by incorporation of the nucleic acid or the sgRNA sequence into the genome of the cell.

Preferably the cells have a single copy of an sgRNA-encoding nucleic acid of the invention per cell. This ensures that only one type of recombinase reaction (either the activation or the inactivation, but not both) happens in a given cell. Different cells may of course have different recombinase reactions - according to the above stochastic principle, thus providing the inactive or active sgRNA populations in cells. To ensure that a cell has only one sgRNA copy, it is possible to target one particular genomic locus, like the AASV1 locus as disclosed in Wang et al. (2014, *supra*), but of course any other unique genetic locus is also possible. Only one insertion into the genome per cell should be possible.

The inventive nucleic acid preferably contains the sgRNA sequence and also preferably a promoter operatively linked to the sgRNA sequence for expression of the sgRNA sequence. The promoter may be a constitutive promoter or an inducible promoter. Especially preferred is a constitutive promoter since activity of sgRNA is regulated by the sgRNA sequence construct of the invention (the guide disruption sequence or the inactivating recombinase product) itself. Example promoters are disclosed, among others, in Ma et al. (Molecular Therapy-Nucleic Acids 3, 2014: e161). The promoter can be an RNA Polymerase II (Pol II) or RNA Polymerase III (Pol III) promoter (see WO2015/099850). Preferably it is a Pol III promoter such as U6, 7SK or H1 promoter. Structures of Pol III promoters are disclosed in Ma et al. 2014. Use of a H1 promoter is e.g. shown in WO2015/195621 (incorporated herein by reference), which methods and construct designs can be used according to any aspect of the invention. A preferred promoter is the U6 promoter.

A Pol II promoter can be selected from the group consisting of retroviral Rous sarcoma virus (RSV) LTR promoter (optionally with the RSV enhancer), the cytomegalovirus (CMV) promoter (optionally with, the CMV enhancer), the SV40 promoter, the dihydro folate reductase promoter, the β-actin promoter, the phosphoglycerol kinase (PGK) promoter, the EFla promoter, and further any one of the CAG, EF1A, CAGGS, PGK, UbiC, CMV, B29, Desmin, Endoglin, FLT-1, GFPA, and SYN1 promoters. Pol II promoters can be used in combination with Csy4 cleavage sites flanking the guide RNA sequence as disclosed in WO2015/099850 or with a selfcleaving ribozyme. The use of pol II in guide expression is further described in WO2015/153940.

The nucleic acid also preferably contains a selection marker. The selection marker can be used to identify and preferably select or isolate cells that contain the inventive nucleic acid. As such successful transformation of a cell with a nucleic acid of the invention can be confirmed and controlled. Cells with the selection marker, and by consequence the inventive sgRNA, may then proceed with the inventive method, step A), etc..

Such a selection marker can be any marker known in the art. It can be a cell survival marker, e.g. an antibiotic resistance gene, or an optical marker, such as a gene encoding a fluorescent protein, like GFP, BFG or RFG.

Preferably the marker is placed in a position that is excised by the first and/or second recombinase activity, i.e. it is flanked by the first and/or second pair of recombinase recognition sites. This removal prevents any hindrance of the selection marker sequence in the formation of an active sgRNA, or in case of the inactive sgRNA, it helps to reduce its size since inactive sgRNAs are preferably identified by sequencing. Smaller sequencing sizes reduce sequencing effort and cost, which is of particular importance in large scale screens when many sgRNA sequences are sequenced. A further advantage of placing the selection marker between each of first and second recombinase recognition site pairs, i.e. in the overlap, is that here it will be removed by both activation and inactivation, which allows counter-selection against premature recombination before using the nucleic acid according to the invention. Accordingly, the "and" option is most preferred, i.e. the marker is preferably flanked by both first and second pairs recombinase recognition sites, i.e. it is within the overlap of the first and second pairs of recombinase recognition site-flanked sequences.

Preferably the inventive nucleic acid contains one or more primer or probe binding site so that nucleic acid primers or probes may bind to the nucleic acid for detection of the inventive sgRNA, either in its non-recombinase-transformed (original) state or in the inactive sgRNA or active sgRNA state. Primers may be used to amplify or sequence the sgRNA sequences for its detection and preferably also identification. Probes can be used to bind the nucleic acid and further probes may be used to bind the sgRNA sequence for its sequence identification.

Preferably the primer or probe binding site is outside the first and second pair of recombinase recognition sites so that it is preserved during and after recombinase action. Such probes or primer binding sites may e.g. flank the guide sequence or the sgRNA sequence its entirety. Preferably two probe or primer binding sites are used, one 5' of the guide sequence or the sgRNA sequence and one 3' of the guide sequence or the sgRNA sequence. The one or more probe or primer binding sites are preferably in the vicinity of the sgRNA sequence, preferably within 20,000 nt (nucleotides) of either end of the sgRNA sequence, preferably within 15,000 nt, or within 10,000 nt, within 5,000 nr or within 1,000 nt, of either end of the sgRNA sequence.

The structure of a sgRNA is for example disclosed in Jiang and Doudna (Annu. Rev. Biophys. 46, 2017:505-29), Swarts et al. (Molecular Cell 66, 2017: 221-233), WO2015/089364, WO2014/191521 and WO2015/065964 and WO 2017/158153 A1. The sgRNA molecule comprises a part that corresponds to the crRNA which contains a guide that is usually 15-30 nt, most often 17-21 nt, in length that mediates target specificity. The crRNA may comprise a pseudoknot structure and/or a seed region. This crRNA part is connected to a part corresponding to the tracrRNA. In the sgRNA, crRNA and tracrRNA parts are fused, usually in a stem-loop region that may contain a (crRNA) repeat, the loop and a (tracrRNA) antirepeat. The stem may contain in addition to palindromic sequences also mismatched nucleotides. The part of the sgRNA corresponding to the tracrRNA may have further loop regions, and generally a 3D folding structure that mediates binding to a Cas enzyme. Inactivating a sgRNA - by action of the recombinase on the second pair of recombinase recognition sites - preferably causes a deletion that prevents binding to a Cas enzyme of choice, such as Cas1, Cas2, Cas3, Cas9, dCas9, Cas10, Cas2a, Cas12b or Cas12c, preferably Cas9 and/or any of its variants, like dCas9, such as by preventing the required fold structure for Cas binding. The deleted region is the region that is flanked by the pair of recombinase recognition sites. Preferably the deletion deletes one or more loops or a part of a loop.

For the active sgRNA, the deletion by recombinase action on the first pair of recombinase recognition sites should maintain an active crRNA-tracrRNA structure and establish the Cas-binding capability of a sgRNA. It is thus preferred that the first recombinase recognition sites - of which one recombinase recognition site remains after recombinase action - is placed in an inert region, such as a loop. Thus, in preferred embodiments of the invention one, preferably two, of the first recombinase recognition sites is located in a loop region of the sgRNA sequence. Preferably, the sgRNA sequence comprises a crRNA part and a tracrRNA part and one of the first recombinase recognition sites is located in a crRNA-tracrRNA linker loop, i.e. a loop that connects the crRNA and tracrRNA parts.

Preferably the sgRNA comprises one or more loops, such as 1, 2, 3, 4, or more loops. One loop is preferably connecting the crRNA-tracrRNA parts. One loop may be contained in the crRNA part, such as in the pseudoknot structure. Preferably the tracrRNA part comprises 1, 2, 3 or more loops that are entirely in the tracrRNA part (not counting the crRNA-tracrRNA linker loop). Usually the first two loops after the crRNA-tracrRNA linker loop are required for Cas binding and one of these, preferably both, is/are deleted or partially deleted (e.g. by deleting one leg of the stem by placing the recombinase recognition site in the loop) for inactivating the sgRNA upon recombinase action. The loops may be connected with stems, such as stems of 3-20 nt in length, wherein the length of one leg of the stem is counted, i.e. the stem may comprise twice its number when counting base pairs - but of course a stem may also comprise base mismatches. Preferably the stem of any one of these loops, especially stemloops entirely of the tracrRNA part, comprise 3-20 base pairs, such as 4 to 15 or 5-10 base pairs, such as 3, 4, 5, 6, 7, 8, 9, 10, 11, 12, 13, 14, 15, 16, 17, 18, 19 or 20 base pairs, preferably 4-7 base pairs. Preferably the crRNA-tracrRNA linker loop is a stem loop with a length of 6, 7, 8, 9, 10, 11, 12, 13, 14, 15, 16, 17, 18, 19 or 20 base pairs in the stem.

The guide disruption sequence, which prevents formation of an active guide in the sgRNA before recombinase action, may comprise a transcription termination sequence, such as a polyA sequence, so that the remainder of the sgRNA is inhibited from transcription. It may also comprise a sequence that prevents an active guideRNA fold or sgRNA fold that is capable to interact with a Cas enzyme to form an active CRISPR-Cas complex. For example, this can be achieved by a sufficient length or inclusion of folding elements that do not bind to the Cas enzyme. The guide disruption sequence may prevent loop formation of any of the loops of the sgRNA, especially preferred the crRNA-tracrRNA linker loop. Such a sequence may e.g. be the selection marker sequence - if it has a sufficient length to prevent an active guideRNA fold. In particular preferred embodiment, both a transcription termination sequence and a sequence (of a length) that prevent the active guideRNA fold are used. In preferred embodiments, the transcriptions termination sequence is placed in a loop, especially preferred the crRNA-tracrRNA linker loop. Preferably also both its flanking first recombinase recognition sites are both placed in the same loop, so that only parts of that loop are deleted in an activating recombinase reaction. In such a case, also one of the second recombinase recognition sites is placed in the same loop since the first and second flanked sequences are overlapping as explained above. Another one of the second recombinase recognition sites is preferably placed downstream, so that essential parts of the sgRNA, especially its tracrRNA are deleted upon recombinase actin on the second pair of recombinase recognition sites. Preferably, a second recombinase recognition site is placed in a loop entirely in the tracrRNA or downstream of the tracrRNA part of the sgRNA, e.g. also downstream of the sgRNA. It may be in a transcribed region or further downstream, such as after a transcription terminus. For example, it may be within 10,000 nt of the 5' end of the sgRNA, preferably within 5,000 nt of the 5' end of the sgRNA.

In preferred embodiments, the first and second pairs of recombinase recognition sites are activated by the same recombinase enzyme. Accordingly, the recombinase may have different recognition sites that to not interact with each other in a recombinase reaction. Such a recombinase is for example Cre. The first and second pairs of recombinase recognition sites may be independently selected from lox sites, such as from loxP, lox 511, lox 5171, lox 2272, M2, M3, M7, M11, lox 71, lox 66.

Using one recombinase for both pairs of recombinase recognition sites has the benefit that only one recombinase needs to be provided to the cell. In other embodiments, the first and second pairs of recombinase recognition sites are activated by different recombinase enzymes. In this case, the population of cells used in an experiment needs to be provided with both recombinase enzymes, wherein an individual cell of that population may have one of both recombinase enzymes, preferably both recombinase enzymes. Example recombinases, for both options (i.e. the same or different recombinases), are site-specific recombinases, such as Cre, Hin, Tre, FLP.

Recombinase reactivity and its selection preference between the first and second pair of recombinase recognition sequences (and thus the stochastic distribution - see above) can be controlled by structural elements and the sequences. A shorter sequence flanked by recombinase recognition sites is preferred over a longer flanked by recombinase recognition sites and will thus lead to more deletion events of its flanked region. Accordingly, the distribution or ratio of active and inactive sgRNAs upon recombinase action can be steered by selecting lengths of flanked regions accordingly. Another option to control distribution or ratio of active and inactive sgRNAs upon recombinase action is to add further recombinase recognition sites, such will also increase its chance to cause a recombinase reaction. It is expected that using more than two recombinase recognition sites will lead to the deletion of the entire sequence portion between the outermost recombinase recognition sites since if a recombinase action would delete a region flanked by an inner recombinase recognition sequence, then still at least two recombinase recognition sequences would remain that lead to a further deletion until only one recombinase recognition site remains. Accordingly, the flanked parts and overlaps should be selected accordingly, such as by considering the outermost recombinase recognition sites as the "pair" of recombinase recognition sites as described herein - even when a "set" of more than two recombinase recognition sites of the same type (as first and/or second sites) is used in the nucleic acid sequence of the invention.

Preferably, the nucleic acid is adapted to provide an average active sgRNA to inactive sgRNA ratio of 9:1 to 1:9, preferably of 5:1 to 1:5, especially preferred of 2:1 to 1:2. Such ratio may also be achieved in the inventive method.

Since recombinase action is length dependent, for proper action of a recombinase the pair of recombinase recognition sites is preferably at most 100,000 nt apart, preferably at most 50,000 nt apart, especially preferred at most 10,000 nt apart or even at most 5,000 nt apart. This applies to the first and/or second pair, preferably both.

Preferably, the inventive nucleic acid contains a unique molecular identifier (UMI) or barcode. A UMI or barcode is a sequence that allows the identification of a particular sgRNA molecule, and is different for each molecule even when targeting the same genetic target (same guide sequence). Example UMIs are random sequences. Such a UMI should have a sufficient length to allow distinction of all nucleic acid molecules used. Preferably the length of the UMI is at least 6 nt, preferably at least 8 nt. For example, it may be 6 to 40 nt in length, preferably 8 to 20 nt in length. Preferably it is placed downstream of the sgRNA sequence. Also preferred, it is located outside of both, the first and second (or any) pair of recombinase recognition sites so that it is not deleted upon recombinase action and preserved in both, active and inactive sgRNAs. Other UMIs may be placed within one (but not the other) of the first and second recombinase recognition sites to allow tracking of only active or only inactive sgRNA (i.e. where it is preserved upon recombinase action). However preferred are the uses of UMIs that are present in both active and inactive sgRNAs. Use of the UMI allows the analysis of independent events passing the bottleneck as independent replicates (Michlits et al., Nature Methods 14, 2017: 1191-1197), so clonal outgrowth can be accounted for. Cells with different UMIs can be used as biological replicates, which are of particular benefit for setups with high heterogeneity in the assay such as organoid cultures and *in vivo* applications. In the inventive methods, therefore, the UMI is used to identify the same sgRNA in different cells. This would mean that these cells are clones of one original cell that has been transformed to contain one particular sgRNA-encoding nucleic acid molecule. The detected UMIs in products after recombinase activation may also indicate the extent of any growth bottlenecks. Small numbers of UMIs per guide in a cell population before and after a bottleneck indicate that cells were lost and to which extent.

The invention also provides cells comprising a nucleic acid encoding the sgRNA of the invention. These cells may be used in the inventive methods. The cells may be mammalian cells, preferably human or non-human cells. If totipotent cells are used, then these are preferably non-human. They may beprimate, murine, bovine, rodent cells. The cells may be isolated cells or cells of an aggregate of cells, such as in a culture, an organoid or *in vivo* cells. *In vivo* cells of the invention are preferably not in a human. The cells may be of a cell line and/or pluripotent cells. However, the cells are not required to remain pluripotent and be allowed to differentiate. Recombinase action (and thus activating a part of the sgRNA according to the stochastic principle) may be done during any time of growth or development. The invention also relates to cells with such an activated sgRNA or inactivated sgRNA.

The cells preferably comprise one or more nucleic acids, like expression constructs, for expression of one or more recombinase(s), such as Cre. The recombinase(s) should be the one that activates the first and/or second recombinase recognition sites as described above. The expression nucleic acids may comprise a selection marker. The section marker may be used to identify and/or isolate cells with active recombinase(s). The selection marker may be a particular sequence, such as a length marker or have a barcode, or contain a cell survival marker, such as an antibiotic resistance gene. A length marker can be identified during sequencing, for example. The marker may alternatively or in addition serve as a control in the production of a nucleic acid encoding the recombinase protein(s), in a virus. To transform cells that should later express recombinase(s) (and be used in the inventive method), it is possible to use a virus as transfection agent. Suitable viruses can be selected with a marker. As mentioned above the nucleic acids, e.g. expression constructs, for expression of the recombinase(s) preferably comprise an inducible or alternatively a constitutive promoter. The recombinase(s) is or are, however, preferably inducible, either by selection of the promoter or by using recombinases that when expressed require activation that can be controlled (e.g. CreER as disclosed above). Also possible is a photoactivatable Cas9 (Nihongaki et al., Nature, 2015, 33(7): 755-760). The inventive method would then also comprise the step of photoactivating Cas. In some cases, the recombinase(s) may not be active in all cells ("non-reacted"). This is usually no problem since a non-recombinase activated sgRNA has a different sequence than the inactive and active sgRNAs after activation and can thus be identified and considered. When using a recombinase under a cell type-specific promoter (e.g. CreER), recombination in fact also selects for cell type specificity and allows to assay only the cell type of interest even if additional cell types were transduced with sgRNAs.

In preferred embodiments of the invention, the sequence of the active/inactive/non-reacted sgRNA is determined after activation/introduction of the recombinase in step B) and preferably after any effects thereof have been observed in the cells after step C). In order to determine the sequence of the sgRNA, preferably the nucleic acid of the invention comprises primer binding sites as mentioned above. Primer binding sites allow sequencing of the sgRNA (including its active/inactive recombination product) and preferably also any UMI if present, whereat the primer binding sites flank the sgRNA sequence and the UMI sequence.

The cells preferably comprise a nucleic acid, like an expression construct, for expression of a Cas, such as Cas9, or any of the above-mentioned Cas enzymes. Also this nucleic acid, e.g. expression construct, for expression of the Cas may comprise an inducible or alternatively a constitutive promoter. An inducible promoter is preferred so that the activity of the Cas enzyme can be controlled. Also the Cas nucleic acid may contain a selection marker, similarly, but selected independently, as described above.

The recombinase and/or the Cas enzyme, preferably both, are provided in the cells. For example, commercial cells are available that have these integrated into their genome. The description of the nucleic acids thus extends to the genome of the cells.

Usually many cells are used in an experiment, such as large-scale screening. Preferably the inventive cells are provided in population of at least 10,000 cells of the invention, more preferred at least 100,000 cells or at least 1 million cells. Preferably the cells have different sgRNAs, such as according to the cells per sgRNA (i.e. sgRNAs with different guides) mentioned above.

The cells may be investigated for any effects of a sgRNA on their growth morphology or activity that may be changed by the active sgRNA in comparison to the cells without the active sgRNA, in particular those with an inactive sgRNA. Such investigated cells may be wild-type cells or carry a mutation. In such cases, the effects of an activated sgRNA on the mutations' effects may be observed. Such a mutation may be an oncogenic mutation, such as an activation or upregulation of an oncogene or a suppression or inactivation of a tumor suppressor gene.

Accordingly, in preferred embodiments of the invention, the cells further express a transgenic oncogene or have a suppressed tumor suppressor gene. The inventive method further comprises the step of overserving differences in tumorigenesis after activation in step C) as compared to cells without the activation in step C), thereby screening for a role of a gene targeted by the sgRNA during tumorigenesis. Parts of the tumor will grow - i.e. the cells with the inactive sgRNA after recombinase action. If no corresponding active sgRNA to an inactive sgRNA is found in the tumor, then the presence of these inactive sgRNAs are evidence that the active sgRNA was initially activated and present but failed to grow in the tumor. Thus, an essential genetic target for tumor growth or its inhibition has been found. As sated above, the presence of the inactive sgRNA provide a proof of absence for the active sgRNAs.

In another embodiment a CRISPR-StAR cassette is integrated in the germline of an animal model or a cell line to enable sparse gene depletion and e.g. generate tumor models with rare, reproducible loss of tumor suppressors. The invention also comprises testing effects of candidate compounds in combination with sgRNA activation. Accordingly, the cells may be further treated with a candidate compound, the method further comprising overserving differences in cell activity or morphology after activation in step C) as compared to cells without the activation in step C), thereby screening for an activity of a gene targeted by the sgRNA under influence of the candidate compound. Such a method may for example be used in a toxicity screen: The candidate compound may be a toxin and the sgRNA may be observed for ameliorating the toxicity when active.

The inventive method is particularly suitable to overcome bottlenecks of low cell counts as discussed before. Such a situation occurs in *in vivo* implantations, in organoids or in heterogenous cell cultures. Accordingly, these are preferred applications of the invention. Accordingly, preferably the cells are grown to or in a tissue aggregate, such as an organoid. The tissue of the aggregate (e.g. tissue of the organoid) may be a liver, spleen, cerebral, muscle, heart, kidney, colorectal, bladder, vascular, ovary, testicular, pancreatic tissue. Also, preferred, the cells are transferred to a non-human animal, preferably to form an allograft or xenograft. The animal is preferably a rodent, non-human primate, cattle, horse, pig, mouse, hamster rat etc. Introduction or activation of the recombinase(s) occurs in the tissue aggregate, organoid, or non-human animal, i.e. after any transplantation or engraftment bottlenecks have passed and the cells of the invention have been preferably grown to a desired number. In other embodiments, the cells are grown in a cell culture, such as a 2D or 3D cell culture. Activation of the recombinase(s) occurs when a desired cell number and/or cell differentiation stage has been reached. Desired cell numbers have been mentioned above with a particular cell/sgRNA ratio.

The invention further provides a kit comprising any means used in the inventive method, like nucleic acids and/or cells. In particular, the kit comprises i) a nucleic acid encoding the sgRNA of the invention and ii) nucleic acid(s) for expression of one or more recombinase(s) that activate(s) the recombinase recognition site pairs of the sgRNA. The kit preferably further comprises iii) a nucleic acid encoding a Cas gene. Any such nucleic acids may be further defined as described above, for example having promoters operatively linked to the sgRNA, recombinase(s), Cas protein. A gene is usually considered to comprise a promoter and a coding region.

The present invention is further illustrated by the following figures and examples, without being limited to these embodiments of the invention.

### Figures

Fig. 1: Distribution of log₂ fold changes between barcodes before and after a pooled CRISPR screen in decreasing numbers of barcodes per guide in library.

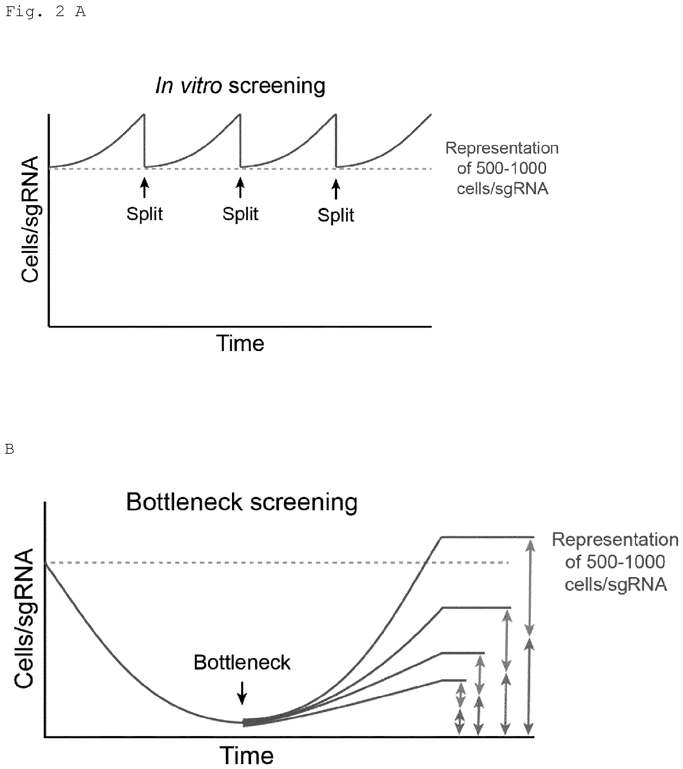

Fig. 2: A) Schematic illustration of 2D *in vitro* genetic screens without bottlenecks; with each split of the cell population the representation of cells/sgRNA is maintained above 500-1,000 cells/sgRNAs to keep the complexity of the screen; B) Schematic illustration of complexity bottlenecks in genetic screens; after a bottleneck caused by infection efficiency, limited cells, engraftment efficiency and/or differentiation cells recover differently, leading to reduced representation of cells/sgRNA. Independent of clone size of cellular heterogeneity, single cell derived clones are stochastically split into an experimental and a control population, depicted as the upper green double arrows (active sgRNAs) and lower red double arrows (inactive sgRNAs).
Fig. 3: (A, B) Schematic representation of CRISPR-StAR vector encoding sgRNAs, stop cassette, selection cassette, tracrRNA and UMIs. Recombination leads to either an active (A) or an inactive (B) sgRNA.
Fig. 4: Schematic illustration of the CRISPR-StAR construct series; StAR1 contains two sets of different lox sites. In comparison to StAR1, StAR3 contains an extra loxP site, a longer distance between the Lox5171 site and the stop cassette and a reduced distance between tracr and the second Lox5171 site. The removal of the extra loxP site resulted in construct StAR4.
Fig. 5: Experimental outline to determine frequency of active to inactive recombination in CRISPR StAR constructs.
Fig. 6: Schematic outline of proof of concept experiment.
Fig. 7: Benchmarking of CRISPR StAR analysis; comparison with traditional day 0 reference.
Fig. 8: Correlation of two biological replicates in high complexities using conventional (active vs day 0) and CRISPR-StAR analysis (active vs inactive). Each dot represents one sgRNA. Density plots and stacked histograms show guide distribution in each replicate. Essentials are shown in red, non-essentials in blue.

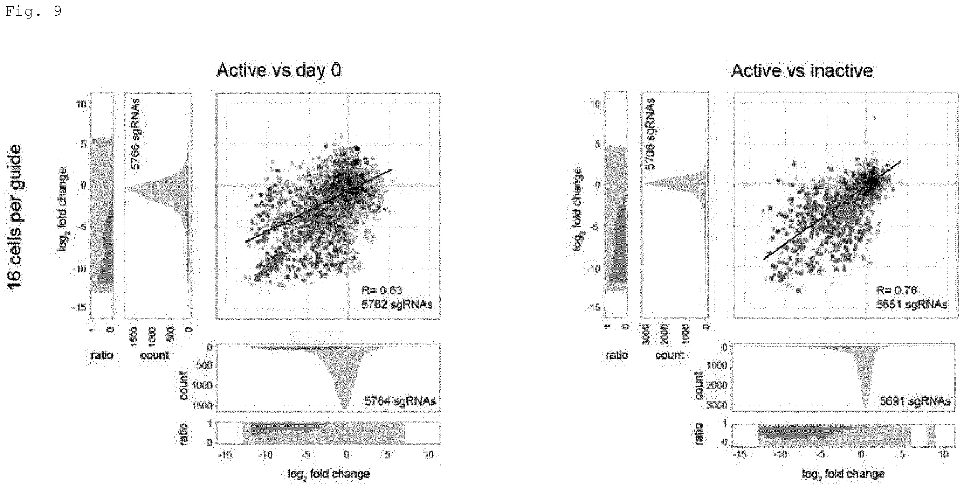

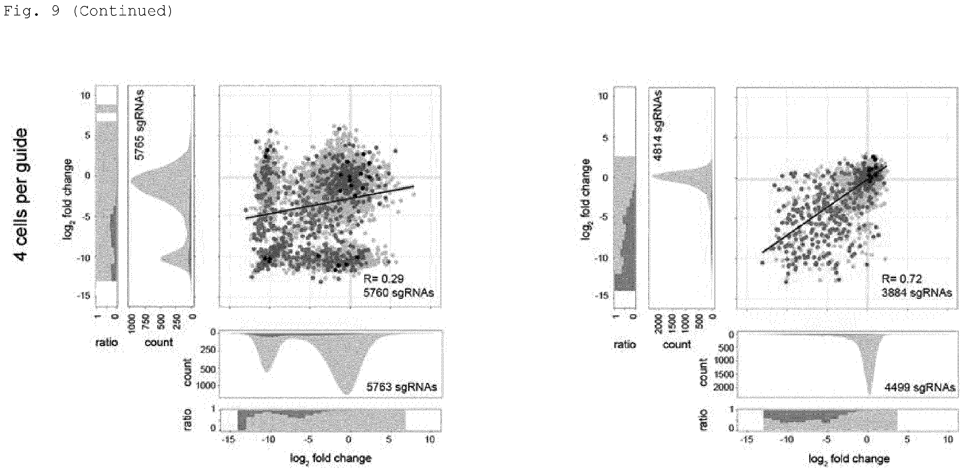

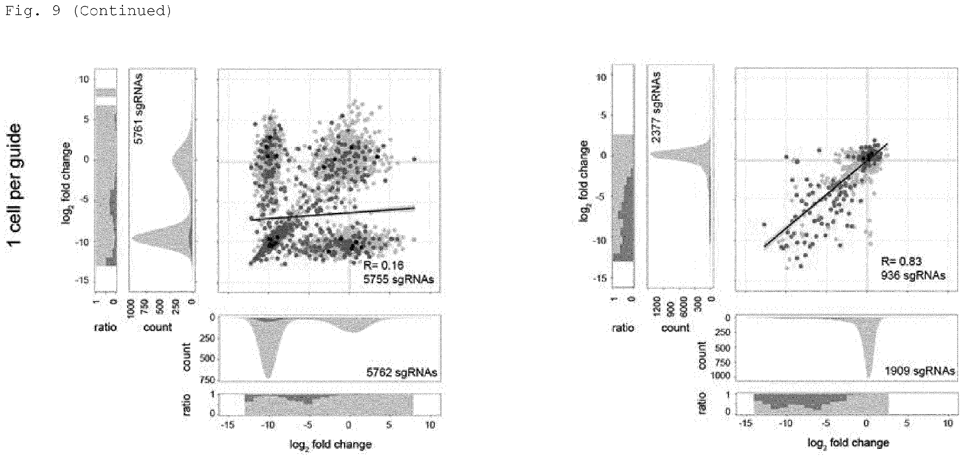

Fig. 9: Correlation of two biological replicates in low complexities using conventional (active vs day 0) and CRISPR-StAR analysis (active vs inactive). Each dot represents one sgRNA. Density plots and stacked histograms show guide distribution in each replicate. Essentials are shown in red, non-essentials in blue. In addition to a dramatically increased spread of neutral (blue) sgRNAs, additional complete dropout is observed at very low representation. This is due to the fact that the sgRNAs were completely lost in the bottleneck. In contrast, CRISPR-StAR only scores sgRNAs that are found in inactive conformation and lost in active.
Fig. 10: Area under the curve analysis of essentials (red) compared to non-essentials (blue) of two biological replicates in decreasing numbers of cells per guide in library.
Fig. 11: Area under the receiver operating characteristic curve (AUROC) analysis of decreasing complexities in cell numbers compared to library. CRISPR StAR analysis (active vs inactive) in green, conventional analysis (active vs day 0) in black
Fig. 12: Pearson correlation, delta area under the curve (dAUC) and area under the receiver operating characteristic curve (AUROC) analysis of decreasing complexities in cell numbers compared to library. Black dots show values of individual replicates, bars show mean of two replicates.
Fig. 13: Improved robustness of organoid screening. a) Correlation of two biological replicates determined by UMI. Density plots and stacked histograms show guide distribution in each replicate. b) The average number of guides targeting the same gene (y-axis) for genes correlated with the top sgRNAs (x-axis), sorted by rank. c) Vulcano plots of conventional (active vs day 0) and CRISPR-StAR analysis (active vs inactive) in two biological replicates determined by UMI. Top genes are shown in blue. Genes that scored in the other replicate are shown in green.

### Examples

### Example 1: Material and methods

### 1.1 Material

### 1.1.1 Cell lines

Tamoxifen-inducible Cre-ERT mouse embryonic stem cells AN3-12 (ESC)
Platinum-E cells (Cell Biolabs RV-101)
Vil-CreERT2; Rosa-LSL-Cas9-2A-eGFP mouse small intestinal organoid

### 1.1.2 Cell culture media

Mouse embryonic stem cell medium (ESCM):
450ml of DMEM, 75ml of FCS (Sigma, 025M3347), 5.5ml of penicillin-streptomycin (Sigma), 5.5ml of NEAA (Gibco), 5.5ml of L-glutamine (Gibco), 5.5ml of sodium pyruvate (Sigma), 0.55ml of β-mercaptoethanol (Merck), 7.5pl of LIF (2mg/ml)

Organoid complete culture medium:
Advanced DMEM/F12, penicillin/streptomycin, 10mmol/L HEPES, Glutamax, 1x N2, 1x B27 (all from Invitrogen), and 1mmol/L N-acetylcysteine (Sigma), recombinant human Wnt-3A, murine EGF, murine noggin, human R-spondin-1, nicotinamide

### 1.1.3 Buffers

Laurylsarcosine lysis buffer:
10mM Tris-HCl pH 7.5 (Sigma Aldrich), 10mM EDTA (Sigma Aldrich), 10mM NaCl (Sigma Aldrich), 0.5% N-laurylsarcosine (Sigma Aldrich), 1mg/ml proteinase K (Thermo Fisher Scientific), 0.1mg/ml RNase A (Qiagen)

2X SDS lysis buffer:
10mM Tris-HCl pH 8 (Sigma Aldrich), 1% SDS (in-house), 10mM EDTA (Sigma Aldrich), 100 mM NaCl (Sigma Aldrich), 0.1mg/ml RNase A (Qiagen)

### 1.1.4 Primers

FW_G_CrSc_5: AATGATACGGCGACCACCGAGATCTACACAGATAACGAGGGCC-
TATTTCCCATGATTCCTTC (SEQ ID NO: 1)
FW_G_CrSc_6: AATGATACGGCGACCACCGAGATCTACACAGCTTGCGAGGGCC-
TATTTCCCATGATTCCTTC (SEQ ID NO: 2)
FW_G_CrSc_7: AATGATACGGCGACCACCGAGATCTACACAGGACACGAGGGCC-
TATTTCCCATGATTCCTTC (SEQ ID NO: 3)
FW_G_CrSc_10: AATGATACGGCGACCACCGAGATCTACACATCACTCGAGGGCC-
TATTTCCCATGATTCCTTC (SEQ ID NO: 4)

FW_G_CrSc_12: AATGATACGGCGACCACCGAGATCTACACCAACACCGAGGGCC-
TATTTCCCATGATTCCTTC (SEQ ID NO: 5)
FW_G_CrSc_13: AATGATACGGCGACCACCGAGATCTACACCACGCCCGAGGGCC-
TATTTCCCATGATTCCTTC (SEQ ID NO: 6)
FW_G_CrSc_15: AATGATACGGCGACCACCGAGATCTACACCATTACCGAGGGCC-
TATTTCCCATGATTCCTTC (SEQ ID NO: 7)
FW_G_CrSc_19: AATGATACGGCGACCACCGAGATCTACACCCCAACGAGGGCC-
TATTTCCCATGATTCCTTC (SEQ ID NO: 8)
FW_G_CrSc_20: AATGATACGGCGACCACCGAGATCTACACCGTCATCGAGGGCC-
TATTTCCCATGATTCCTTC (SEQ ID NO: 9)
FW_G_CrSc_21: AATGATACGGCGACCACCGAGATCTACACCTATGCCGAGGGCC-
TATTTCCCATGATTCCTTC (SEQ ID NO: 10)
FW_G_CrSc_22: AATGATACGGCGACCACCGAGATCTACACCTCCGCCGAGGGCC-
TATTTCCCATGATTCCTTC (SEQ ID NO: 11)
FW_G_CrSc_39: AATGATACGGCGACCACCGAGATCTACACTGCCGACGAGGGCC-
TATTTCCCATGATTCCTTC (SEQ ID NO: 12)
FW_G_CrSc_41: AATGATACGGCGACCACCGAGATCTACACTGTAGACGAGGGCC-
TATTTCCCATGATTCCTTC (SEQ ID NO: 13)
FW_G_CrSc_42: AATGATACGGCGACCACCGAGATCTACACTTGCCACGAGGGCC-
TATTTCCCATGATTCCTTC (SEQ ID NO: 14)
RV_G_CrSc: CAAGCAGAAGACGGCATACGAGATACCGTTGATGAGTAG (SEQ ID NO:
15)
NGS_U6: CGATTTCTTGGCTTTATATATCTTGTGGAAAGGACGAAACACCG (SEQ ID NO:
16)

### 1.2 Methods

### 1.2.1 Mouse embryonic stem cell culture

Cells were cultured in ESCM, which was changed daily. When confluent, cells were trypsinized and split 1:10. For 4-hydroxytamoxifen (4OH) treatment, medium was supplemented every day with 0.5pM 4OH (Sigma).

### 1.2.2 Small intestinal organoid culture

Intestinal organoids were established from a Vil-CreERT2; Rosa-LSL-Cas9-2A-eGFP (homozygous) mouse. For organoid establishment, crypts were isolated from the mouse small intestinal epithelium after washing and dissociation. Isolated crypts were resuspended in Matrigel (Corning) at a density of 150-200 crypts per 20 µl droplet. Droplets were seeded in 48-well plates (Corning) and 250 µl of media was used in each well. For the first two passages, cells were cultured in complete organoid medium supplemented with Rho-kinase inhibitor (Y- 27632, R&D Systems). Organoids were split every 5-7 days through mechanical pipetting in 1:5 to 1:6 ratios.

### 1.2.3 Single cell derived clones

ESCs were trypsinized and counted. 500 cells were seeded on a 15 cm dish (Sigma Aldrich). ESCM was exchanged every 2 days. Colonies were allowed to grow for 10 days, then picked into 96-U well plates (Thermo Fisher), trypsinized and split onto 96-F well plates (Thermo Fisher). Cells were cultured until confluent, lysed with 75 µl Laurylsarcosine lysis buffer at 37°C overnight. For amplification, 1 µl lysate was used in 25 µl PCR reactions (95°C 3min, [95°C 20sec, 65°C (-0.3 °C per cycle) 20sec, 72°C 30sec] x 23, [95°C 20sec, 58°C 20sec, 72°C 30sec] x 30, 72°C 3min, 12°C ∞).

### 1.2.4 Retroviral vectors and ESC infection

The CRISPR-StAR library was packaged into Platinum-E cells according to the manufacturer's recommendations. 300 million ESCs were infected with a 1:10 dilution of virus-containing supernatant in the presence of 2 pg/ml polybrene. 24 hours after infection, selection for infected cells was started with blasticidin and puromycin at 1 pg/ml each. To estimate the multiplicity of infection, 10,000 cells were plated on 15 cm dishes and selected with G418. For comparison, an additional 1,000 cells were plated and were not exposed to G418 selection. On day 10, colonies were counted.

### 1.2.5 Cell culture screen

ESCs were infected with a retroviral CRISPR-StAR vector, selected for blasticidin and puromycin resistance for 3 days. To mimic bottlenecks, cells were thoroughly counted and seeded in densities of 1 cell/sgRNA (5870 cells), 4 cells/sgRNA, 16 cells/sgRNA, 64 cells/sgRNA, 256 cells/sgRNA, 1024 cells/sgRNA in the library. Over the course of 7 days cells were grown to equal densities. To induce recombination, ESCs were treated with 5 µM 4OH for 3 days. They were maintained for another 14 days.

### 1.2.6 Organoid screen

To prepare for the screen, organoids were expanded in 10 cm dish format (Corning). In each 10 cm dish, 50-55 droplets were seeded and each droplet containing around 100 organoids and in total ten 10 cm dishes were used in the screen. Each dish was supplemented with 10 ml of complete medium and refreshed every two days. To prepare organoids for viral infection, organoids were first mechanically broken down to small pieces. After spin down (500g x 5 min) and removing the supernatant (which contains old Matrigel), cells were resuspended in TrypLE (Gibco) and dissociated to 5- to 8-cell clumps at 37°C. Cells were spun down at 300g for 3 min. After removing the supernatant, cell pellets were resuspended in virus-containing media and dispensed into 48-well plates. The plate was sealed with parafilm and spinoculation was performed for 1 h at 37°C. After spinoculation, parafilm was removed and the plate was incubated at 37°C for 6 h. Afterwards, cells were transferred to Eppendorf tubes and spun down (300g x 3 min). The cell pellet was resuspended in Matrigel and seeded onto 10 cm dishes. After 3 days of recovery, infected organoids were selected for blasticidin resistance for 8 days at 1 pg/ml. Subsequently, organoids were dissociated, and complete medium was substituted with 4OH for 6 h. Afterwards, organoids were kept in culture in complete medium for 12 days without splitting. Medium was refreshed every 3 days.

### 1.2.7 DNA harvest and NGS sample preparation

60 million cells per sample were collected and lysed in SDS lysis buffer plus 1 mg/ml Proteinase K and 0.1 mg/ml RNAseA. Genomic DNA was extracted with phenol and chlorophorm and precipitated with 1 volume isopropanol. The integrated sgRNA construct is flanked by PacI restriction sites. Samples were digested with PacI for 48 h and co-digested with BbsI for the last 12 h. Each sample was PCR amplified in 96 individual 50 µl reactions with 1 µg DNA per reaction (95°C 3 min, [95°C 10 sec, 59°C 20 sec, 72°C 30 sec] x 36, 72°C 3min, 4°C ∞). Forward primers were unique for each sample and contained a 6 bp experimental index for demultiplexing after NGS (AATGATACGGCGACCACCGAGATCTACAC-NNNNNN-CGAGGGCCTATTTCCCATGATTCCTTC (SEQ ID NO: 17), where the 6-bp NNNNNN sequence represents specific experimental indices used for demultiplexing samples after NGS). Reverse primer was the same for each sample. PCR products were purified and size-separated by agarose gel electrophoresis. The two recombination products were excised separately, purified on a mini-elute column and mixed in equal amounts. This sample was sequenced on an Illumina HiSeqV4 SR100 dual-indexing sequencing run. sgRNAs were sequenced with a custom read primer. To distinguish active from inactive guide, the sequence downstream of the first lox site (either TCAGCATAGC for active or TTTTTTT for inactive) was chosen.

### Example 2: Concept Overview

In genetic screens, genome editing can have three major effects: it can give a growth benefit, a growth disadvantage or have no effect to cells targeted with a specific sgRNA. A growth benefit will lead to enrichment within the population. A growth disadvantage will lead to depletion.

Pooled CRISPR screens are usually kept at a complexity of 300-1,000 individually targeted cells per sgRNA. This allows a sufficient number of unique editing events to call a significant change in the population. However, it is not always possible to maintain this high level of complexity. When a system encounters a bottleneck caused by inefficient infection or limited cell numbers or differentiation or if cells recover at different rates and the library representation decreases. To illustrate this, we calculated log₂ fold changes (LFC) between read numbers of barcodes before and after a CRISPR screen. The numbers of barcodes represent the numbers of differently transformed cells, i.e. the numbers of barcodes per guide represent the numbers of cells/sgRNA.

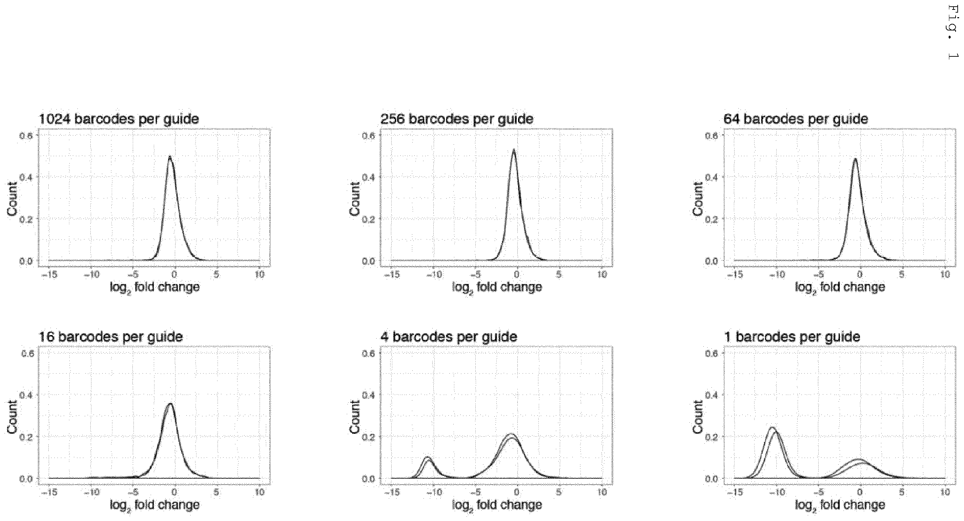

As complexity decreases, the distribution in LFC becomes broader because fewer barcodes are present and changes in the population have larger effects. When complexity further decreases, the distribution becomes bimodal with appearance of a second peak with strong LFC (Figure 1). This peak is due to missing guides with 0 reads. In analysis, these guides will be mistaken for guides causing a strong depletion phenotype and therefore skew screening results. This means that with insufficient complexity, read numbers of guides before the screen are no longer comparable to read numbers after the screen and conventional analysis fails.

The problems caused by insufficient library representation upon bottlenecks in CRISPR screens can be overcome by the invention (illustrated in Figure 2).

### Example 3: sgRNA constructs

Due to two sets of interlaced lox sites, the CRISPR StAR system can give rise to two different recombination products: an inactive sgRNA or an active sgRNA. The vector contains an sgRNA (library), followed by two pairs of lox sites in the tracr region. Between the lox sites there is a blasticidin selection cassette to prevent premature activation due to e.g. Cre activity or recombination events during viral packaging. Lastly, it contains a stretch of random nucleotides acting as unique molecular identifiers (UMIs). Recombination of the loxP sites results in an active sgRNA (Figure 3A), whereas recombination of the lox5171 sites results in termination and exclusion of the tracr. As a consequence, the sgRNA is inactive (Figure 3B). The two recombination events are mutually exclusive.

With this system, it is possible to compare active guides to an inactive internal control within the final population of a CRISPR screen. However, it is beneficial to compare of read numbers of the two recombination products, if the ratio of active to inactive recombination is fairly similar. For most cases, the ratio of loxP (active) to lox5171 (inactive) recombination should be between 10:90 and 90:10.

Recombination probability between the one and the other loxP pair depends on several factors such as distance and DNA structure (primary, secondary, and tertiary) at the locus. It is therefore difficult to predict. Single cell quantification of recombination probabilities revealed, that the original construct (StAR1) resulted in a recombination ratio of 33% active sgRNAs to 66% inactive sgRNAs. Such ratio is ideal if screens desire to monitor relative enrichment of active over inactive sgRNAs, as it offers an ideal dynamic range. However, for the analysis of essential genes, it is preferable to start with equal ratio of active sgRNAs relative to inactive or even a bias towards active sgRNAs. We therefore developed StAR3 and StAR4 by modification or relative distances, primary sequence, and introduction of one additional loxP site (Figure 4). In doing so, we successfully generated a series of constructs resulting in different recombination ratios:

| | Active | Inactive |
|---|---|---|
| StAR1 (SEQ ID NO: 18): | 33% | 66% |
| StAR3 (SEQ ID NO: 19): | 90% | 10% |
| StAR4 (SEQ ID NO: 20): | 50% | 50% |

Depending on the desired experiment, different setups will be ideal.

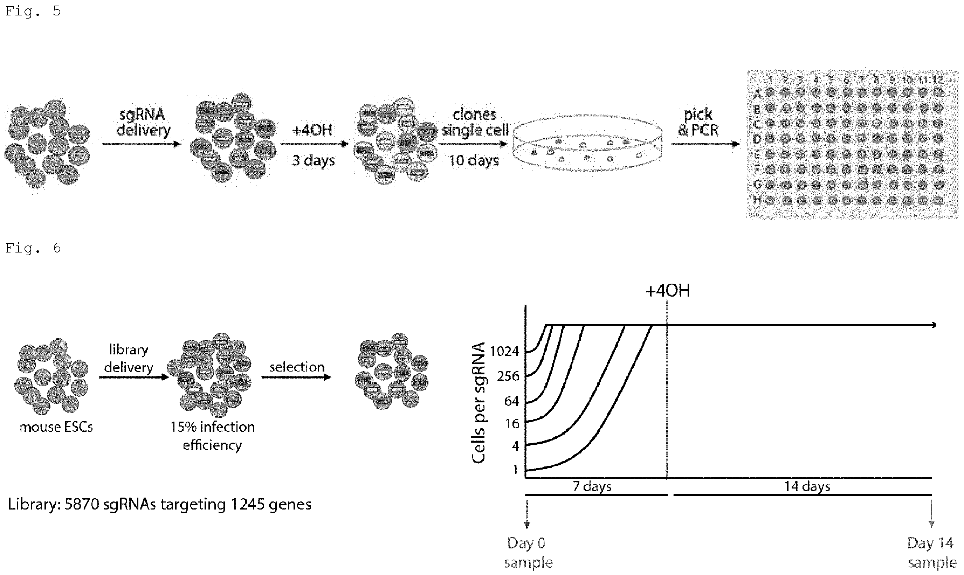

To determine how efficient either pair of lox sites recombines, sgRNA-infected cells were treated with 4OH for 3 days and subsequently seeded in clonal density (Figure 5). At this point, recombination has happened and these clones either expressed an active or an inactive guide. To identify them, we did PCR with primers flanking the guide construct. Recombination products are 580 bp for active or 542 bp for inactive. We counted frequency of each band size. Most importantly, we found no unrecombined clones, which confirms stable Cre expression in our cell line. The above recombination frequencies were found. For StAR1, out of 288 total clones, recombination resulted in 97 active and 172 inactive sgRNAs. We found 21 double bands which are either due to contaminated mixed clones or double infections. They were counted for both events.

### Example 4: Cell culture

### 4.1 Experimental design

To confirm that CRISPR-StAR overcomes noise in bottleneck screens, we introduced controlled bottlenecks in a cell culture experiment. Therefore, we infected mouse embryonic stem cells with stable integration of a Cas9 expression cassette as well as a CreERT2 expression construct with a retroviral sgRNA StAR1-type library of 5,870 sgRNAs targeting 1,245 genes (Table 1).

**Table 1: Library subpools**

| | **Genes** | **Guides** |
|---|---|---|
| Drugable genes | 885 | 4453 |
| Handpicked | 360 | 1417 |
| **Sum** | **1245** | **5870** |

15% of cells were infected to ensure single infections. After selection for viral integration, we counted and diluted the cells to introduce controlled bottlenecks. Complexity was reduced to 1 cell/sgRNA (5,870 cells), 4 cells/sgRNA, 16 cells/sgRNA, 64 cells/sgRNA, 256 cells/sgRNA, 1,024 cells/sgRNA. Cells were grown to equal densities of more than 1,000 cells/sgRNA over the course of 7 days. Subsequently, cells were treated with 4OH to induce Cre recombination and cells were maintained for another 14 days. The experiment was executed in 2 independent replicates (Figure 6).

After 14 days, genomic DNA was extracted and digested with PacI using cut sites flanking the construct. We then amplified the guide construct via PCR from the fragmented genome with primers containing experimental indices and Illumina adaptors for each sample, which allowed direct sequencing of the PCR product. We gel-extracted both recombination products separately and mixed them in a 1:1 ratio. This pool was then sequenced.

### 4.2 Bioinformatic pipeline

After mapping NGS reads, we used the 10 bp stretch directly downstream of the first loxP site to bioinformatically distinguish active from inactive guides (either TCAGCATAGC for active or TTTTTTT for inactive). Although active and inactive recombination products were mixed in a 1:1 ratio before sequencing, we found twice more reads from inactive than from active guides, which indicates that inactive constructs sequence better. Nevertheless, analysis does not suffer from this situation.

Each cell was infected with a single guide construct. Thus, every UMI represents one clone and the number of UMIs per guide is equal to the number of cells per guide, which in turn is a direct measure of how many cells per guide were infected. To check whether cell dilutions in the proof of concept experiment were sufficient, we calculated median number of UMIs per inactive guide in lowest complexity samples (1 cell per guide). However, instead of the theoretical 1 UMI per guide, we found much higher numbers. We hypothesized two reasons for this: First, most of these UMIs had only one or two reads, which is most likely due to base substitution errors in sequencing; second, when we calculated distribution of read numbers per UMI, we found a bimodal distribution. When we looked at the sgRNA-UMI combinations from the low read fraction of this distribution, we could find the same sgRNA-UMI combinations with high read numbers in different samples. This suggested index hopping, which is a known problem in Illumina based sequencing, where indices between neighboring clusters are assigned to the wrong sample. In higher complexity samples these issues are negligible because there are high numbers of true UMIs per guide, so overall, these errors have a very small impact. Therefore, this is only relevant in lower complexity samples (1-16 cells per sgRNA). Here, true reads have a distinct distribution with high read counts, while the errors have a distribution with low reads.

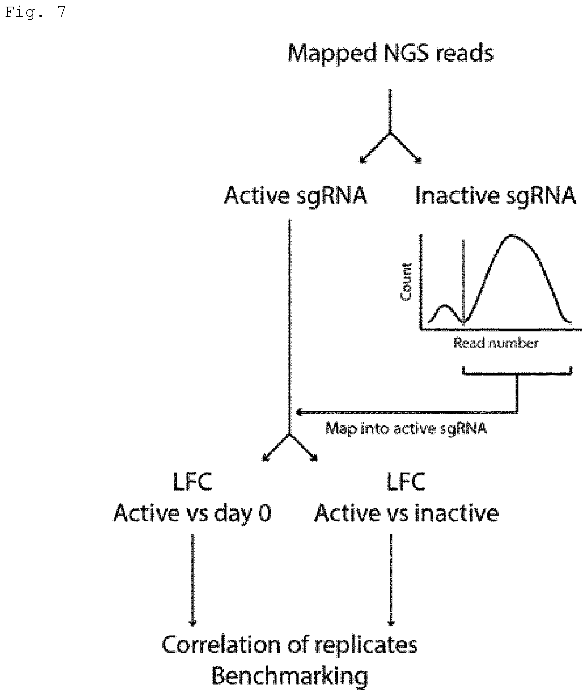

To separate true reads from errors, we defined the local minimum in this bimodal read distribution for each low complexity sample as a threshold and discarded all reads below. Since the read number of an UMI in an active guide can represent a phenotype, we only set a cutoff in inactive guides and mapped the sgRNA-UMI combination in the active guides, which further cleaned the dataset of non-existing UMIs.
Finally, to benchmark performance of CRISPR StAR to conventional CRISPR screen analysis, we calculated LFC for both methods: active guides versus day 0 for conventional analysis as well as active versus inactive guides for CRISPR-StAR analysis (Figure 7) .

### 4.3 Benchmarking

To benchmark performance of CRISPR-StAR compared to conventional screening methodology, we calculated Pearson coefficients between replicates, delta area under the curve (dAUC) and area under receiver operating characteristic curves (AUROC).

### 4.3.1 Replicate correlation

To test reproducibility of our results, we calculated correlation coefficients between two biological replicates on essential and non-essential guides. In order to do this, we defined essential genes (red) using data of two independent screens performed in the same cell line, with the same library at a high complexity. We calculated median depletion of each guide and defined guides with a LFC lower than -3 as essential. On the other hand, we defined non-essentials (blue) as the same number as essentials of the least depleting guides from the same dataset. We then correlated LFCs of guides in two independent replicates and determined Pearson coefficients based on essentials and non-essentials. To get a better understanding of data distribution, we calculated densities and ratios of essentials and remaining data for each replicate (Figures 8 and 9, side density plots). Lastly, we counted number of sgRNAs present in each replicate as well as overlap between both replicates.

At high complexities of 64-1,024 cells per sgRNA, with both conventional and CRISPR StAR analysis, we found good correlation between replicates. Although distribution of data is slightly broader using conventional analysis than using CRISPR StAR, essentials can clearly be separated from non-essentials. Correlation coefficients range from 0.72 to 0.75 using conventional analysis and from 0.80 to 0.84 with CRISPR-StAR (Figure 8). In this homogeneous system, 64 cells per sgRNA seems to be a sufficient complexity for CRISPR screening using conventional analysis.

Using conventional analysis in lower complexities of 1-16 cells per sgRNA, we found an increased spread of both essential and non-essential guides. In 4 and 1 cells per sgRNA samples, the distribution of data becomes bimodal. This is due to sgRNAs with 0 reads in either one or both replicates that cause a strong depletion when compared to day 0. This depletion can either be due to a phenotype caused by a guide, or it can be due to the absence of the guide in the final population. Especially in systems that encounter bottlenecks, it is likely that guides get lost. With conventional analysis, it is not possible to distinguish missing guide from a phenotype. In contrast, when using CRISPR StAR analysis, abundance of active guides is compared to abundance of inactive control guides within the final population. Therefore, guides that got lost due to the effect of a bottleneck will be excluded from analysis. The resulting guide population is smaller and LFCs are due to a phenotype caused by a guide. As a result, in the lowest complexity sample (1 cell per sgRNA), using conventional analysis, correlation decreases to 0.16, while with CRISPR StAR analysis with 0.83 it is as high as in the most complex sample (Figure 9).

In conclusion, using conventional analysis we found poor reproducibility with decreasing complexities. This is due to an increased spread of data caused by missing guides. Using CRISPR StAR, missing guides are removed, and only present guides are considered. Therefore, results are highly reproducible even at low complexity.

### 4.3.2 dAUC

Calculating dAUC of defined categories within a population gives a measure of how well members of each category can be separated from one another. Using this, we benchmarked performance of CRISPR StAR against conventional analysis in separating essentials from non-essentials. For this, we subset essential and non-essential guides, as defined above, to a new list and ranked them by LFC from most depleting to most enriching. We then calculated the cumulative fraction for occurrence of each guide in a category throughout the ranked list. In other words, if an essential guide scores, the essential curve goes up. The same is true for non-essentials. If the guides have an effect, essentials must be ranked on the top of the list, which results in rapid increase, followed by a plateau, where no essentials are scored. On the other hand, non-essentials are ranked at the end of the list and this is represented by a plateau followed by a rapid increase. Ideally, we would expect both categories to be clearly separated from one another. Therefore, the better method will show a better separation. To get a comparable measure, we calculated dAUC by subtracting AUC of essentials from AUC of non-essentials. An ideal score, if all essentials are separated from non-essentials would be 0.5. A random sample would result in a diagonal line and the dAUC score would be 0.

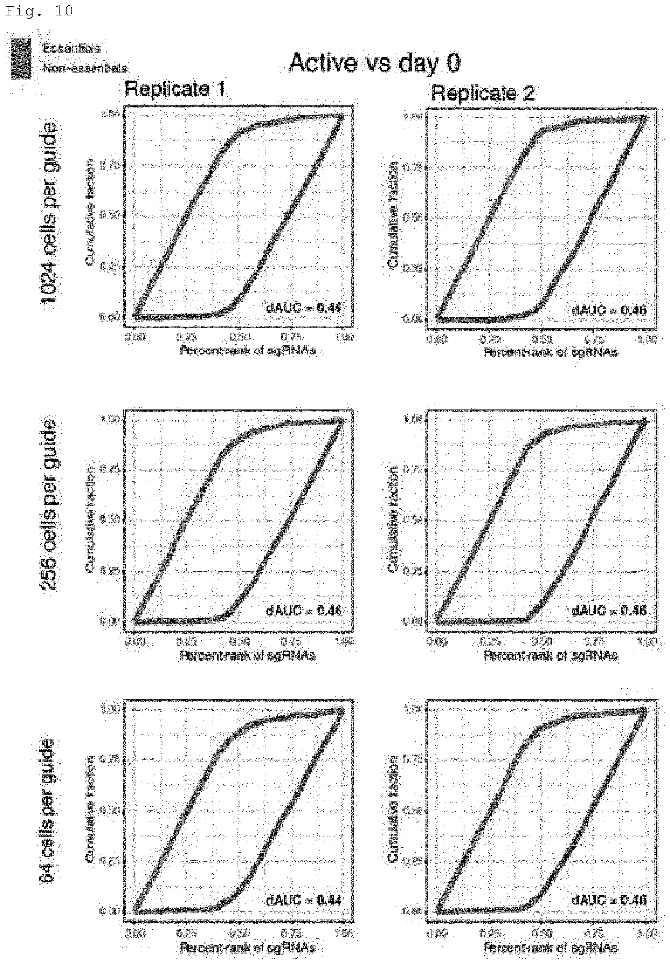

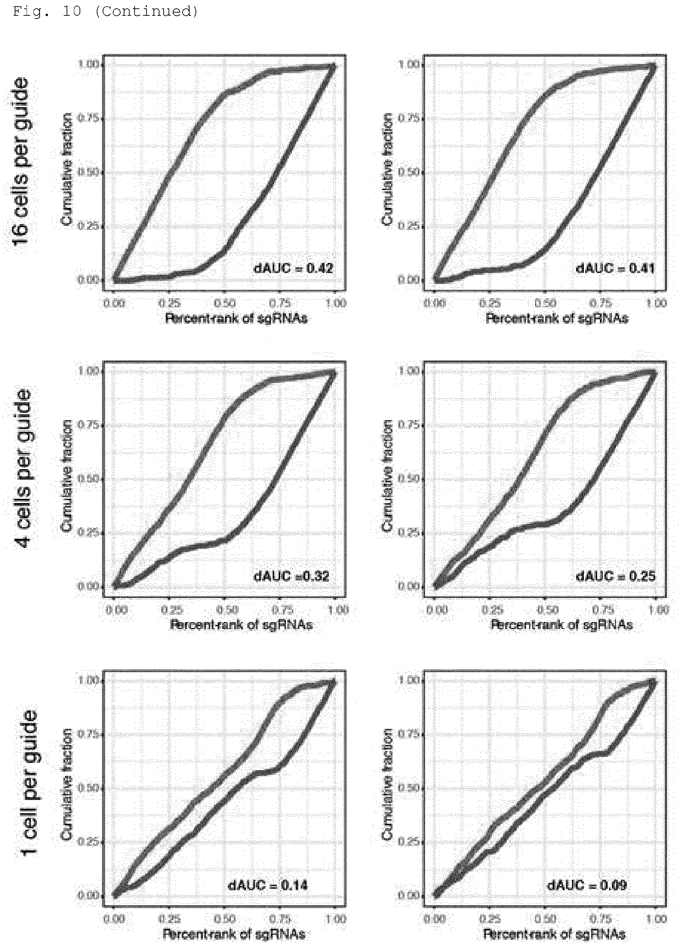

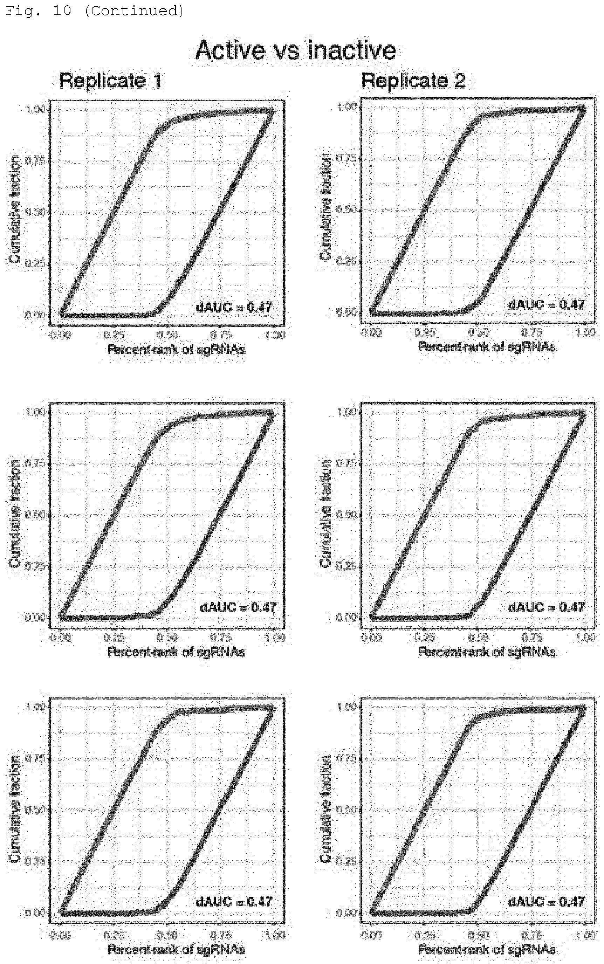

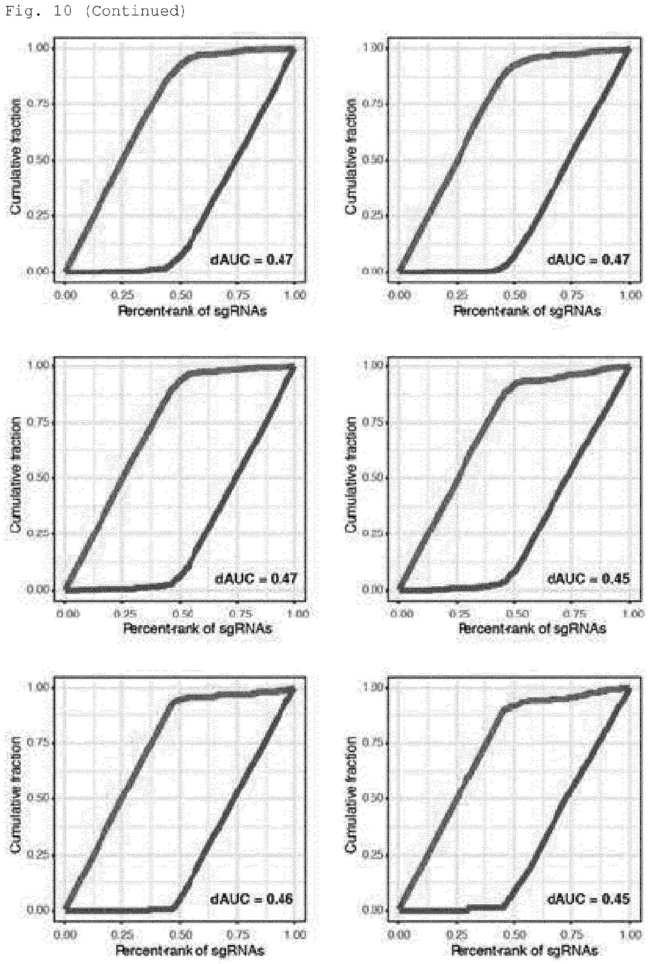

The dAUC for CRISPR-StAR analysis is stably ranging from 0.45 to 0.47. Even in the lowest complexity samples dAUC are 0.46 and 0.45, respectively. In contrast, using conventional analysis, with decreasing complexity, essentials can no longer be cleanly separated from non-essentials. As above, this is caused by a broad spread of both essentials and non-essentials (Figure 9). dAUC drops to 0.14 and 0.09 in the lowest complexity samples, respectively (Figure 10). Therefore, CRISPR StAR analysis outperforms conventional analysis by clearly identifying essentials as essential and by separating them from non-essentials.

### 4.3.3 AUROC

In receiver operating characteristic (ROC) curves, true positive rates are compared to false positive rates. They quantify how well a method can classify data, in this case: guides, into essentials or non-essentials. We defined essentials as above and categorized them as true positives. In the same manner, we categorized non-essentials as false positives. We calculated AUROC scores on a ranked list of guides by LFC for true CRISPR StAR and conventional analysis using the pROC package in R. An ideal score would be 1, a random score would be 0.5.

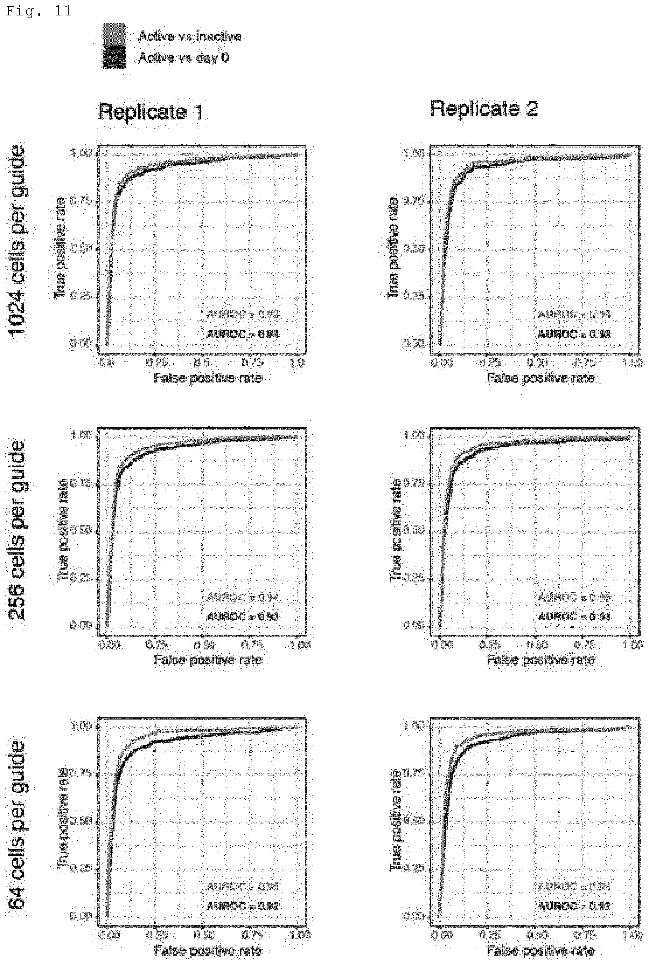

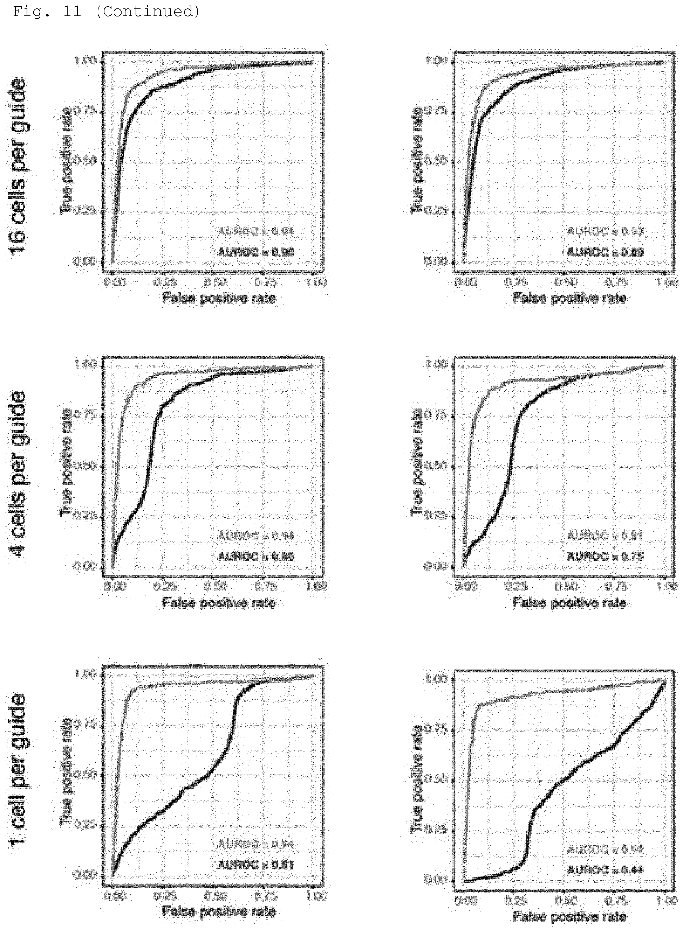

For conventional analysis, with decreasing complexity, AUROC drops from 0.94 to 0.44, which is the same as a random score (Figure 11). Non-essentials that deplete are absent from analysis. This causes a large LFC, which scores them wrongly as essentials. In contrast, with CRISPR StAR analysis, the AUROC remains between 0.91 and 0.95. Therefore, even at the lowest complexity, true positives can clearly be distinguished from false positives.

### 4.4 Summary

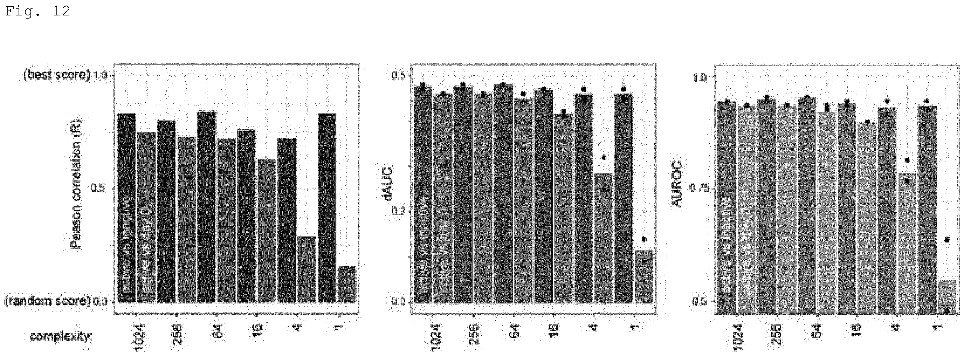

We calculated Pearson coefficients, dAUC and AUROC to benchmark performance of CRISPR StAR against conventional CRISPR screen analysis. Using all three methods we found that with decreasing complexities CRISPR StAR clearly outperforms conventional analysis especially in the lowest complexity samples (Figure 12).

Taken together, the presented data confirm that CRISPR-StAR indeed overcomes noise in genetic screens that is introduced by the loss of complexity after bottleneck in screening population.

### Example 5: Organoid screen

In homogeneous cell populations, conditions that support high resolution CRISPR screening can be easily controlled. In more heterogeneous systems such as organoids, this is a major difficulty. To specifically test the effect of clonal heterogeneity in a model, we tested CRISPR-StAR in intestinal organoids. First, our retroviral library delivery will only infect the stem cells in the crypt, which is a small subset of the whole cell population. Therefore, infection in organoids is very inefficient and usually represents the first bottleneck that needs to be overcome. Secondly, clonal outgrowth is very heterogeneous.

We transduced organoids carrying CreERT2 and Cas9 transgenes with our sgRNA library. They were selected for blasticidin resistance for 8 days, treated with 4OH-tamoxifen to induce Cre recombination and kept in culture for another 12 days.

To estimate the complexity of infection, we calculated median number of UMIs per guide. Similar to the cell culture screen, we saw a bimodal read distribution caused by index swapping. We handled this in the same way we did in the cell culture screen; i.e. to separate true reads from errors, we defined the local minimum in this bimodal read distribution as a threshold and discarded all reads below. Since the read number of an UMI in an active guide can represent a phenotype, we only set this cutoff in inactive guides and mapped the sgRNA-UMI combination in the active guides, which further cleaned the dataset of non-existing UMIs. After the cutoff, we found that infection occurred at a complexity of 30 cells per sgRNA.

UMIs on the guide construct allow for tracking of clonal outgrowth of individually marked cells, thus every UMI within the same guide represents a biological replicate. Thus, we modified our dataset by splitting it into two groups according to first letter of UMI: UMIs starting with A or T in one group and UMIs starting with C and G in another. These two groups were then used as biological replicates.

### 5.1 Benchmarking

To benchmark performance of CRISPR-StAR in organoids compared to conventional screening methodology, we calculated Pearson coefficients between replicates based on UMIs. Next, we analyzed guide reproducibility within a ranked list of guides by calculating the number of genes compared to the number of guides, and scored correlation of two biological replicates determined by UMI. Lastly, we compared hit lists in both types of analysis within the same two replicates.

### 5.2 Correlation

To compare reproducibility of CRISPR StAR to conventional analysis, we calculated Pearson coefficients between these UMI-based biological replicates. To generate a day 0 sample for conventional analysis, we took both replicates of day 0 samples in the proof of concept screen and calculated mean read numbers of each guide. As we do not know the complete essentialome of organoids, we could not apply the same benchmarking procedure as for the cell culture screen (Example 2.2). Instead, we used core essentials as defined by Hart (Hart et al., Cell 163(6), 2015: 1515-1526) that should be depleting in every cell type.

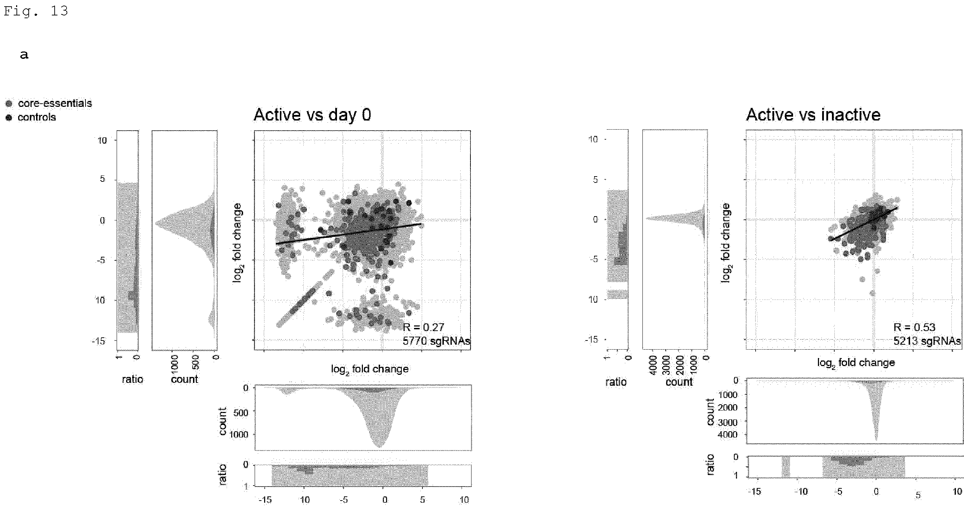

We found a poor reproducibility of screening results using conventional analysis (R=0.27) while CRISPR-StAR analysis of the same dataset generated more reproducible hit lists (R=0.53). Overall, the spread of data is larger when using conventional analysis. In contrast, there is a very sharp signal with CRISPR StAR analysis, after identifying 557 missing guides, which were lost in the bottleneck, and were therefore excluded from CRISPR StAR analysis (Figure 13a).

### 5.3 Guide reproducibility

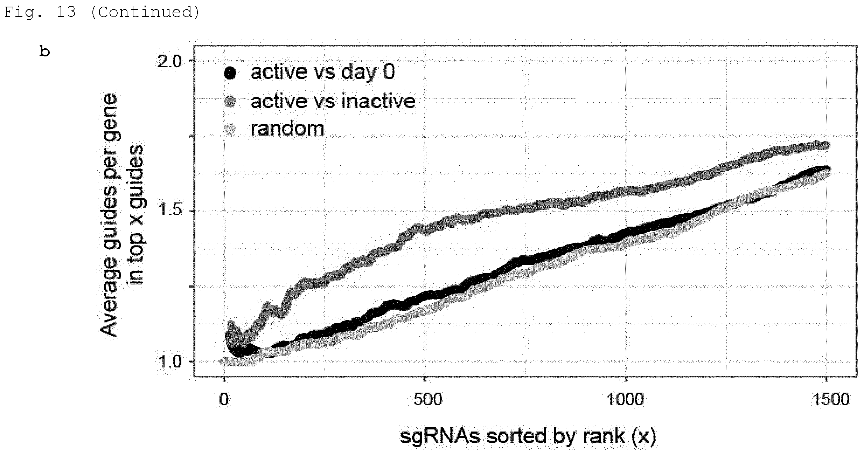

To test guide reproducibility, we used MAGeCK algorithm to generate a ranked list of guides. From this list, we calculated the average number of sgRNAs present per gene for all genes hit by the respective group of guides sorted by rank. For example, if 15 genes hit within the top 30 sgRNAs, the value was 2; a value of 1 would be expected for a random data set. While conventional analysis leads to a close to random result, CRISPR-StAR shows higher reproducibility of scored genes (Figure 13b).

### 5.4 Gene reproducibility

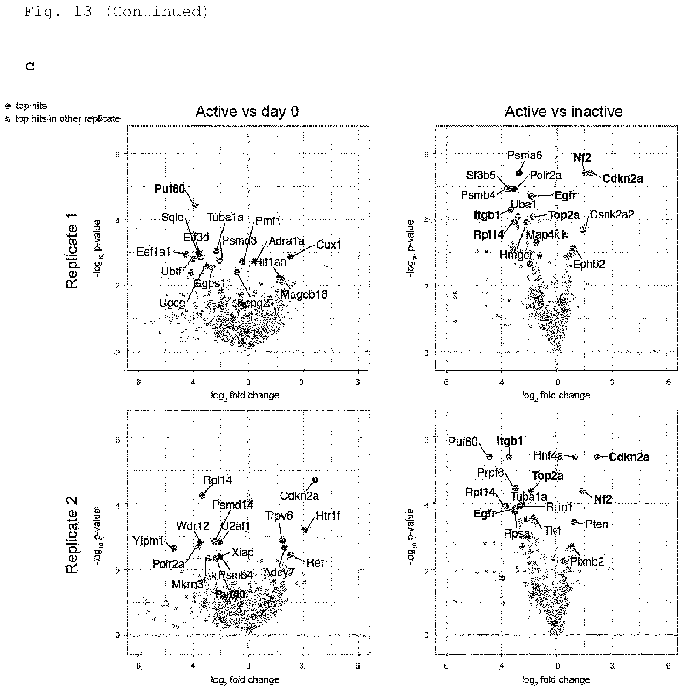

Lastly, for comparison at gene level, we used MAGeCK for both ways of analysis to combine guides and create a ranked list of genes. Not only could we call top hits with higher p-values compared to conventional analysis, but the scored genes were also more reproducible between replicates. Furthermore, using CRISPR-StAR analysis, out of the top 10 depleting genes we called 4 out of the top 10 depleting genes in both replicates, n contrast to only one commonly depleting gene using conventional analysis. These are hits that we expect to find since they are either core essential or specific to organoid growth (Egfr, Itgb1, Top2a, Rpl14). Under the top 5 enriching genes, we found 2 that were common between replicates (Nf2, Cdkn2a), while we did not find any common genes using conventional analysis (Figure 13c). Furthermore, genes that scored in the respective other replicate are scoring highly in CRISPR-StAR analysis, while they are rather distributed in conventional analysis.

We conclude that CRISPR-StAR can identify screen hits robustly and thereby outperforms conventional analysis, allowing reproducible results even in heterogeneous systems such as intestinal organoids.

## Claims

1. A nucleic acid comprising a sequence encoding a single guide RNA (sgRNA) of a CRISPR/Cas system, wherein the sgRNA sequence is interrupted by a guide disruption sequence flanked by a first pair of recombinase recognition sites, and wherein the sgRNA sequence further comprises a second pair of recombinase recognition sites that has a different recombinase recognition sequence than the first pair of recombinase recognition sites, wherein the guide disruption sequence is not flanked by the second pair of recombinase recognition sites and/or wherein the second pair of recombinase recognition sites flank a part of the sgRNA required to form an active sgRNA; and wherein the sequences flanked by the first and second recombinase recognition sites overlap.

2. The nucleic acid of claim 1, wherein one recombinase recognition site of the second recombinase recognition site pair is located between the first pair of recombinase recognition sites and preferably downstream of the guide disruption sequence, and another recombinase recognition site of the second recombinase recognition site pair is located downstream of the first pair of recombinase recognition sites.

3. The nucleic acid of claim 1 or 2, wherein one of the first recombinase recognition sites is located in a loop region of the sgRNA sequence, preferably wherein the sgRNA sequence comprises a crRNA part and a tracrRNA part and one of the first recombinase recognition sites is located in a crRNA-tracrRNA linker loop.

4. The nucleic acid of any one of claims 1 to 3, wherein the guide disruption sequence comprises a transcription disruption sequence or has sufficient length to prevent folding into an active sgRNA fold.

5. The nucleic acid of any one of claims 1 to 4, wherein the first and second pairs of recombinase recognition sites are activated by the same recombinase enzyme, preferably they are independently selected from lox sites, even more preferred from loxP, lox 511, lox 5171, lox 2272, M2, M3, M7, M11, lox 71, lox 66.

6. The nucleic acid of any one of claims 1 to 5, further comprising a selection marker sequence, preferably an antibiotic selection marker sequence, which is preferably located between the pairs of recombinase recognition sites, especially preferred between both the first and second pairs of recombinase recognition sites.

7. A method of expressing an sgRNA of the CRISPR/Cas system upon recombinase stimulation, comprising
A) providing a plurality of cells with a plurality of nucleic acids of any one of claims 1 to 6,
B) introducing or activating one or more recombinases in the cells that are capable of activating the first and second recombinase recognition site pairs,
C) wherein activation of the first recombinase recognition site pair and of the second recombinase recognition site pair are competing reactions, wherein activation of the first recombinase recognition site pair leads to expression of an active sgRNA and wherein activation of the second recombinase recognition site pair inactivates the sgRNA sequence.

8. The method of claim 7, wherein the cells of the plurality have a single copy of a nucleic acid of claims 1 to 6 per cell.

9. The method of claim 7 or 8, wherein the cells are multiplied; after step A) and before step B), preferably wherein the cells are multiples to a number of at least 250 cells per number of different sgRNA sequences in the plurality of nucleic acids of any one of claims 1 to 6.

10. The method of any one of claims 7 to 9, wherein cells with the inactive part of the sgRNA sequence are identified to detect the presence of a sgRNA sequence.

11. The method of any one of claims 7 to 10, wherein the cells further express a transgenic oncogene or have a suppressed tumor suppressor gene, the method further comprising overserving differences in tumorigenesis after activation in step C) as compared to cells without the activation in step C), thereby screening for a role of a gene targeted by the sgRNA during tumorigenesis; or wherein the cells are further treated with a candidate compound, the method further comprising overserving differences in cell activity or morphology after activation in step C) as compared to cells without the activation in step C), thereby screening for an activity of a gene targeted by the sgRNA under influence of the candidate compound.

12. The method of any one of claims 7 to 11, wherein the nucleic acid of any one of claims 1 to 6 comprises a unique molecular identifier (UMI) sequence, wherein the UMI is used to identify the same sgRNA in different cells.

13. The method of any one of claims 7 to 12 wherein the cells comprise a nucleic acid sequence for expression of a recombinase, preferably Cre, wherein said nucleic acid for expression of a recombinase preferably also comprises a selection marker.

14. A cell comprising a nucleic acid of any one of claims 1 to 6.

15. A kit comprising i) a nucleic acid of any one of claims 1 to 6 and ii) one or more nucleic acids for expression of one or more recombinases that is/are capable to activate both recombinase recognition site pairs of the nucleic acid of any one of claims 1 to 6.
